# EUROPEAN PATENT SPECIFICATION

(11) **EP 1 082 341 B1**
(45) Date of publication and mention of the grant of the patent: **08.12.2010**
(21) Application number: 99927164.6
(22) Date of filing: 01.06.1999
(51) Int. Cl.: C07K 14/00

(54) **TUMOR ANTIGEN USEFUL IN DIAGNOSIS AND THERAPY OF PROSTATE AND COLON CANCER**
TUMORANTIGEN VERWENDBAR IN DER DIAGNOSE UND THERAPIE VON PROSTATA UND DICKDARM-KREBS
NOUVEL ANTIGENE TUMORAL UTILE DANS LE DIAGNOSTIC ET LA THERAPIE PROPRES AU CANCER DE LA PROSTATE ET DU COLON

(30) Priority: 01.06.1998 US 87598 P; 29.06.1998 US 91474 P; 14.04.1999 US 129521 P
(43) Date of publication of application: 14.03.2001
(73) Proprietor: Agensys, Inc., Santa Monica CA 90404 (US)
(72) Inventor: AFAR, Daniel, E., Brisbane, CA 94005 (US); HUBERT, Rene, S., Los Angeles, CA 90026 (US); LEONG, Kahan, Playa Del Rey, CA 90293 (US); RAITANO, Arthur, B., Los Angeles, CA 90064 (US); SAFFRAN, Douglas, C., Los Angeles, CA 90049 (US); MITCHELL, Stephen Chappell, Santa Monica, CA 90405 (US)
(74) Representative: Campbell, Patrick John Henry
(86) International application number: PCT/US1999/012253
(87) International publication number: WO 1999/062942

(56) References cited:
- WO-A-93/01309
- WO-A-94/16083
- PAOLONI-GIACOBINO A. ET AL., : "Cloning of the TMPRSS2 gene, which encodes a novel serine protease with transmembrane, LDLRA, and SRCR domains and maps to 21q22.3." GENOMICS , vol. 44(3), 1997, page 309-320 XP000856785 cited in the application
- DATABASE EMBL NUCLEOTIDE AND PROTEIN SEQUENCES,11 March 1997 (1997-03-11), XP002132696 HINXTON, GB
- DATABASE EMBL NUCLEOTIDE AND PROTEIN SEQUENCES,20 May 1997 (1997-05-20), XP002132697 HINXTON, GB
- FAIR W R ET AL., : "Prostate-specific membrane antigen." PROSTATE, vol. 32 (2), 1997, page 140-148 XP000870112
- YOUNG C Y F ET AL.,: "Expression and androgenic regulation of human prostate-specific kallikreins" JOURNAL OF ANDROLOGY, vol. 16, no. 2, April 1995 (1995-04), pages 97-99, XP000856769 cited in the application
- TANIMOTO H T AL., : "Hepsin, a cell surface serine protease identified in hepatoma cells, is overexpressed in ovarian cancer" CANCER RESEARCH, vol. 57 (14), 1997, page 2884-2887 XP000867297 cited in the application
- YAMAMOTO M ET AL., : "Raised prostate-specific antigen in adenocarcinoma of the colon" INTERNATIONAL UROLOGY AND NEPHROLOGY , vol. 29 (2), 1997, page 221-225 XP000889898
- DATABASE EMBL NUCLEOTIDE AND PROTEIN SEQUENCES,16 November 1998 (1998-11-16), XP002132700 HINXTON, GB

## Description

### BACKGROUND OF THE INVENTION

Prostate cancer is the most frequently diagnosed cancer and second leading cause of cancer death in men. Some 45,000 men die annually of this disease. Only lung cancer has a higher mortality. The chance of a man developing invasive prostate cancer during his lifetime is 1 in 6. At the age of 50, a man has a greater than 40% chance of developing prostate cancer and nearly a 3% chance of dying from this disease. While some advances in the treatment of locally confined tumors have been achieved, prostate cancer is incurable once it has metastasized. Patients with metastatic prostate cancer are treated by hormonal ablation therapy, but with only short-term success. Eventually, these patients develop an androgen-refractory state leading to disease progression and death.

A continuing and fundamental problem in the management of prostate cancer is the absence of reliable diagnostic and prognostic markers capable of accurately detecting early-stage localized tumors and/or predicting disease susceptibility and progression. Early detection and diagnosis of prostate cancer currently relies on digital rectal examination (DRE), prostate specific antigen (PSA) measurements, transrectal ultrasonography (TRUS), and transrectal needle biopsy (TRNB). Serum PSA measurements in combination with DRE represent the leading diagnostic approach at present. However, this approach has major limitations which have fueled intensive research into finding better diagnostic markers of this disease. A number of markers have been identified, and at least one, PSA, is in widespread clinical use. However, ideal prostate tumor markers have been extremely elusive and no marker has yet proven reliable for predicting progression of the disease. Thus, there is a need for more reliable and informative diagnostic and prognostic methods in the management of prostate cancer.

In addition, there is also great interest in identifying prostate-specific proteins that could be appropriate as therapeutic targets, as there is no effective treatment for patients who develop recurrent disease or who have been diagnosed with metastatic disease. Although hormone ablation therapy can palliate these patients, the majority inevitably progress to develop incurable, androgen-independent disease (Lalani et al., 1997, Cancer Metastasis Rev. 16: 29-66).

PSA is the most widely used tumor marker for screening, diagnosis, and monitoring prostate cancer today. In particular, several immunoassays for the detection of serum PSA are in widespread clinical use. Recently, a reverse transcriptase-polymerase chain reaction (RT-PCR) assay for PSA mRNA in serum has been developed. However, PSA is not a disease-specific marker, as elevated levels of PSA are detectable in a large percentage of patients with BPH and prostatitis (25-86%)(Gao et al., 1997, Prostate 31: 264-281), as well as in other nonmalignant disorders and in some normal men, a factor which significantly limits the diagnostic specificity of this marker. For example, elevations in serum PSA of between 4 to 10 ng/ml are observed in BPH, and even higher values are observed in prostatitis, particularly acute prostatitis. BPH is an extremely common condition in men. Further confusing the situation is the fact that serum PSA elevations may be observed without any indication of disease from DRE, and vice-versa. Moreover, it is now recognized that PSA is not prostate-specific (Gao et al., supra, for review).

Various methods designed to improve the specificity of PSA-based detection have been described, such as measuring PSA density and the ratio of free vs. complexed PSA. However, none of these methodologies have been able to reproducibly distinguish benign from malignant prostate disease. In addition, PSA diagnostics have sensitivities of between 57-79% (Cupp & Osterling, 1993, Mayo Clin Proc 68:297-306), and thus miss identifying prostate cancer in a significant population of men with the disease.

Prostate-Specific Membrane Antigen (PSMA) is a recently described cell surface marker of prostate cancer which has been the subject of various studies evaluating its use as a diagnostic and therapeutic marker. PSMA expression is largely restricted to prostate tissues, but detectable levels of PSMA mRNA have been observed in brain, salivary gland, small intestine, and renal cell carcinoma (Israeli et al., 1993, Cancer Res 53: 227-230). PSMA protein is highly expressed in most primary and metastatic prostate cancers, but is also expressed in most intraepithelial neoplasia specimens (Gao et al., supra). Preliminary results using an Indium-111 labeled, anti-PSMA monoclonal antibody to image recurrent prostate cancer show some promise (Sodee et al., 1996, Clin Nuc Med 21: 759-766). PSMA is a hormone dependent antigen requiring the presence of functional androgen receptor. Since not all prostate cancer cells express androgen receptor, the clinical utility of PSMA as a therapeutic target may be inherently limited. Clinical trials designed to examine the effectiveness of PSMA immunotherapy are also underway.

Prostate Stem Cell Antigen (PSCA) is another very recently described cell surface marker of prostate cancer (Reiter et al., 1998, Proc. Natl. Acad. Sci. USA 95: 1735-1740). PSCA expression has been shown to be prostate specific and widely over-expressed across all stages of prostate cancer, including high grade prostatic intraepithelial neoplasia (PIN), androgen-dependent and androgen-independent prostate tumors. The PSCA gene has been mapped to chromosome 8q24.2, a region of allelic gain in more than 80% of prostate cancers. PSCA shows promise as a diagnostic and therapeutic target in view of its cell surface location, prostate specificity, and greatly upregulated expression in prostate cancer cells.

Progress in the identification of specific markers has been has been slow due to a lack of experimental animal model systems that recapitulate clinical disease. Attempted solutions to this problem have included the generation of prostate cancer cell lines (Horoszewicz et al., 1983, Cancer Res. 43, 1809) and prostate cancer xenografts (Pretlow et al., 1991, Cancer Res. 51, 3814; van Weerden et al., 1996, Am. J. Pathol. 149, 1055; Klein et al., 1997, Nature Med. 3, 402). However, these approaches have met with limited success. For example, xenografts have generally produced low long-term survival rates. In addition, none of the most widely used human prostate cancer cell lines - PC-3, DU-145, and LNCaP - have been shown to reproducibly give rise to osteoblastic lesions typical of prostate cancer. A further limitation of the DU-145 and PC-3 cell lines is that these cells do not express prostate specific antigen (PSA) or androgen receptor (AR) (Kaighn et al., 1979, Invest. Urol. 17: 16-23; Gleave et al., 1992, Cancer Res. 52: 1598-1605), questioning their relevance to clinical prostate cancer. The LNCaP cell line is androgen responsive and expresses PSA, but contains a mutation in the androgen receptor which alters ligand specificity.

Recently, however, a series of prostate cancer xenografts (derived from patient tumors) demonstrating genetic and phenotypic characteristics closely paralleling the human clinical situation have been described (Klein et al., 1997, Nature Med. 3: 402). These LAPC (Los Angeles Prostate Cancer) xenografts have survived passage in severe combined immune deficient (SCID) mice for longer than one year. The LAPC-4 xenograft model system has the capacity to mimic the transition from androgen dependence to androgen independence (Klein et al., 1997, supra). LAPC-4 tumors regress in male mice after castration, but re-grow within 2-3 months as androgen independent tumors. Both androgen dependent (AD) and androgen independent (AI) LAPC-4 xenograft tumors express equal levels of the prostate specific markers PSA, PSMA (prostate specific membrane antigen) and PSCA (prostate stem cell antigen), which was identified using representational difference analysis of cDNAs derived from the AD and AI variants of the LAPC-4 xenograft.

### SUMMARY OF THE INVENTION

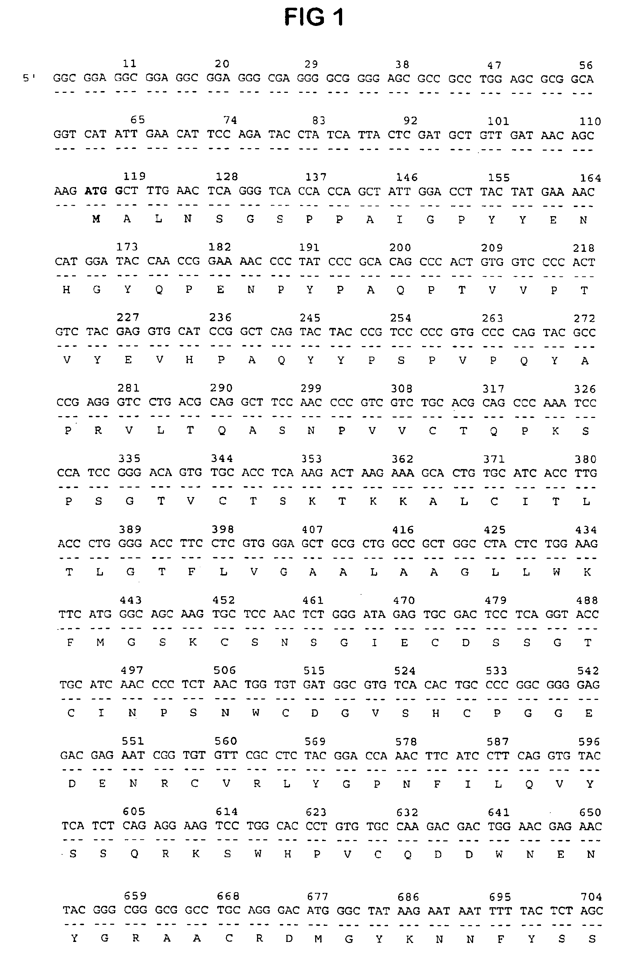

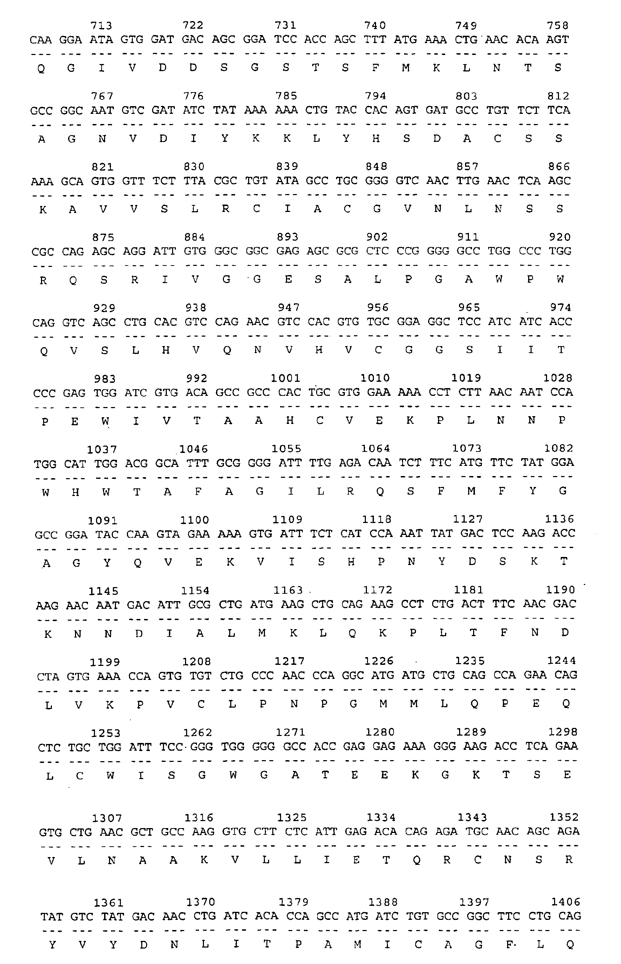

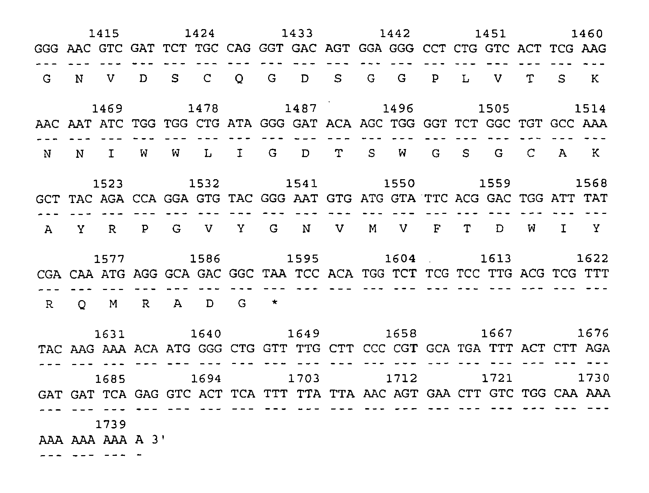

The present invention relates to methods and compositions for the diagnosis and therapy of prostate and colon cancer, derived from or based on a novel prostate-specific, androgen-regulated, cell surface serine protease termed 20P1F12/TMPRSS2 and extensively described herein. A full length cDNA comprising the entire coding sequence of the 20P1F12/TMPRSS2 gene (also designated 20P1F12-GTC1 herein) is provided (FIG. 1). This cDNA encodes a protein which is highly related to, but structurally distinct from, the recently published TMPRSS2 (Paoloni-Giacobino et al., 1997 Genomics 44: 309-320). The 20P1F12/TMPRSS2 gene also shows a very different expression pattern relative to the expression profile of TMPRSS2.

In accordance with one aspect of the present invention, there is provided an isolated 20P1F12/TMPRSS2 protein comprising the amino acid sequence shown in FIG. 1. In another aspect of the present invention, there is provided an isolated polynucleotide selected from (a) a polynucleotide having the nucleotide sequence as shown in FIG. 1, wherein T can also be U; (b) a polynucleotide encoding a 20P1F12/TMPRSS2 polypeptide whose sequence is encoded in the cDNA contained in plasmid p20P1F12-GTC1 as deposited with American Type Culture Collection as Accession No. 207097; and (c) a polynucleotide encoding the 20P1F12/TMPRSS2 protein of FIG. 1.

Recombinant DNA molecules containing 20P1F12/TMPRSS2 polynucleotides, cells transformed or transduced with such molecules, and host-vector systems for the expression of 20P1F12/TMPRSS2 gene products are also provided.

In another aspect, the present invention provides an assay for determining the presence of prostate or colon cancer in an individual, comprising contacting a test sample of prostate or colon tissue with an antibody that immunospecifically binds to the protein consisting of the amino acid sequence of FIG. 1, and which antibody is labeled with a detectable marker, and detecting the binding of 20P1F12/TMPRSS2 protein in the sample thereto, wherein a significant increase in 20P1F12/TMPRSS2 protein expression in the test tissue sample relative to expression levels in the corresponding normal tissue indicates the presence of prostate or colon cancer.

In another aspect, the invention provides an assay for determining the presence of prostate or colon cancer in an individual, comprising:
(a) contacting a sample of prostate or colon tissue with a polynucleotide probe which specifically hybridizes to the polynucleotide of Fig. 1; and
(b) detecting the presence of a hybridization complex formed by the hybridization of the probe with 20P1F12/TMPRSS2 polynucleotide in the sample, wherein the presence of the hybridization complex indicates the presence of 20P1F12/TMPRSS2 polynucleotide within the sample, wherein a significant increase in 20P1F12/TMPRSS2 polynucleotide in the sample relative to levels in a corresponding normal tissue indicates the presence of cancer.

In another aspect, the invention provides an assay for determining the presence of prostate or colon cancer in an individual, comprising:
(a) producing cDNA from a prostate or colon tissue sample by reverse transcription using at least one primer;
(b) amplifying the cDNA so produced by using sense and antisense primers to amplify 20P1F12/TMPRSS2 cDNAs therein; and
(c) detecting the presence of the amplified 20P1F12/TMPRSS2 cDNA,
wherein the sense and antisense primers are capable of amplifying the polynucleotide of Fig. 1, and wherein a significant increase in 20P1F12/TMPRSS2 mRNA or cDNA in the test tissue sample relative to levels in a corresponding normal tissue indicates the presence of prostate or colon cancer.

The invention also provides an antibody or antigen binding fragment thereof that immunospecifically binds to the protein consisting of the amino acid sequence of Fig. 1, for use in a method for the treatment of prostate cancer. Antibodies that bind to the protein of FIG. 1 include polyclonal and monoclonal antibodies, murine and other mammalian antibodies, chimeric antibodies, humanized and fully human antibodies, and antibodies labeled with a detectable marker or toxin or therapeutic composition. Several monoclonal antibodies specifically reactive with 20P1F12/TMPRSS2 are also described herein. These and other 20P1F12/TMPRSS2 antibodies are useful in molecular diagnostic assays and diagnostic imaging methods for detecting, localizing and characterizing carcinomas of the prostate and colon and metastases thereof.

### BRIEF DESCRIPTION OF THE FIGURES

FIG. 1. Nucleotide and deduced amino acid sequences of cDNA clone 20P1 F12-GTC1 (Example 3)(as deposited with the ATCC; Accession No. 207097).

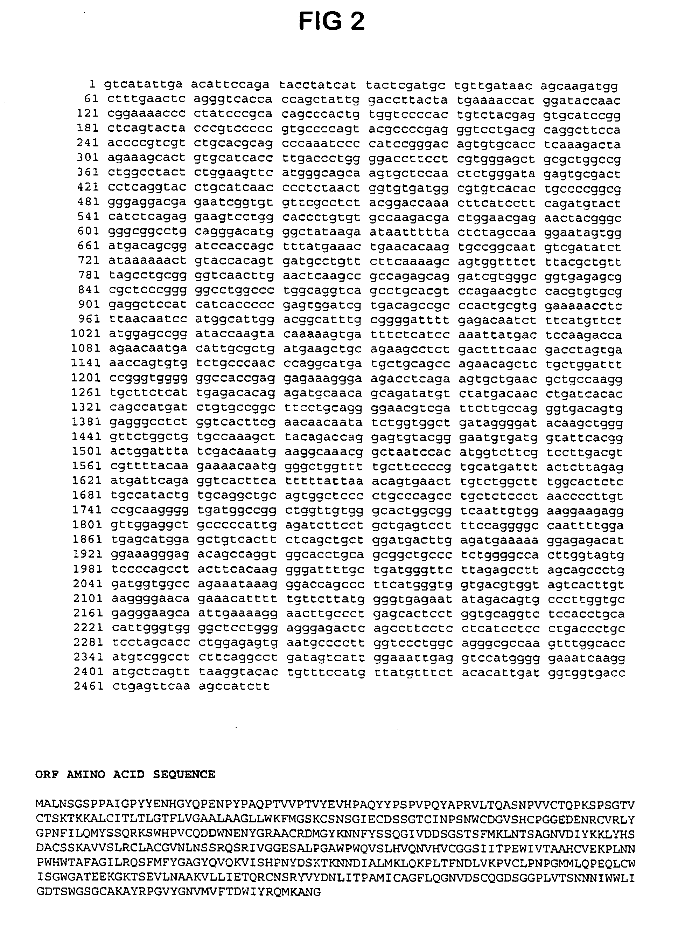

FIG. 2. Nucleotide and deduced amino acid sequences of TMPRSS2 gene sequence as published in Paoloni-Giacobino et al., 1997, Genomics 44: 309-320.

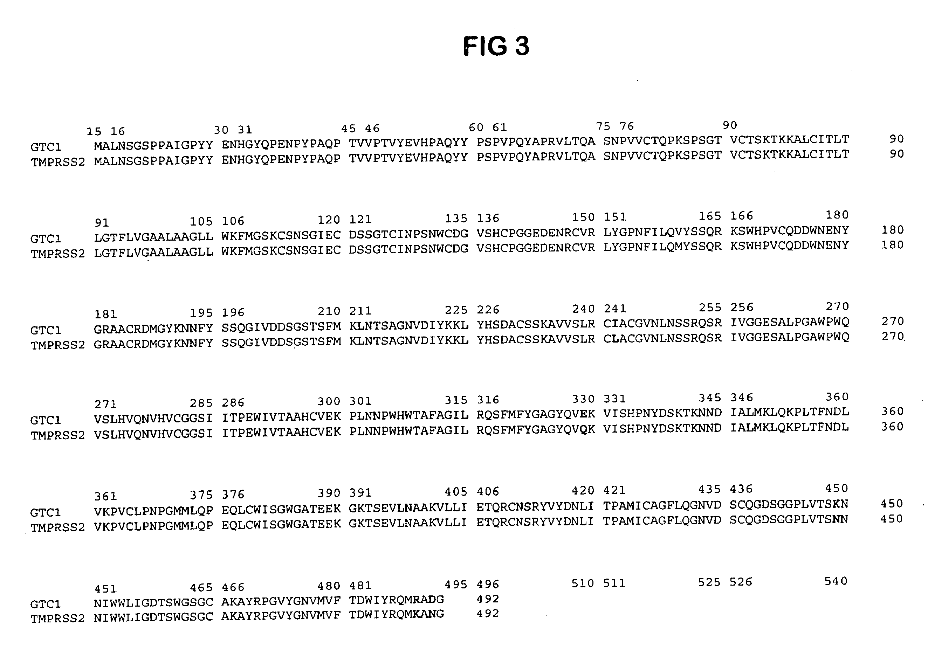

FIG. 3. Amino acid sequence alignment comparing 20P1F12-GTC1 cDNA (Example 3) with the previously published sequence of TMPRSS2 (Paoloni-Giacobino et al., 1997, Genomics 44: 309-320). Amino acid differences are shown in bold type.

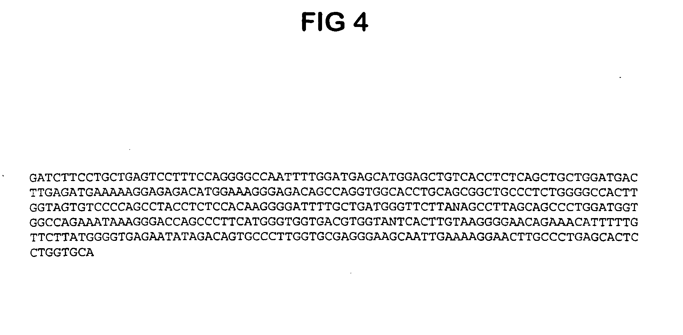

FIG. 4. Nucleotide sequence of initially isolated SSH clone 20P1 F12.

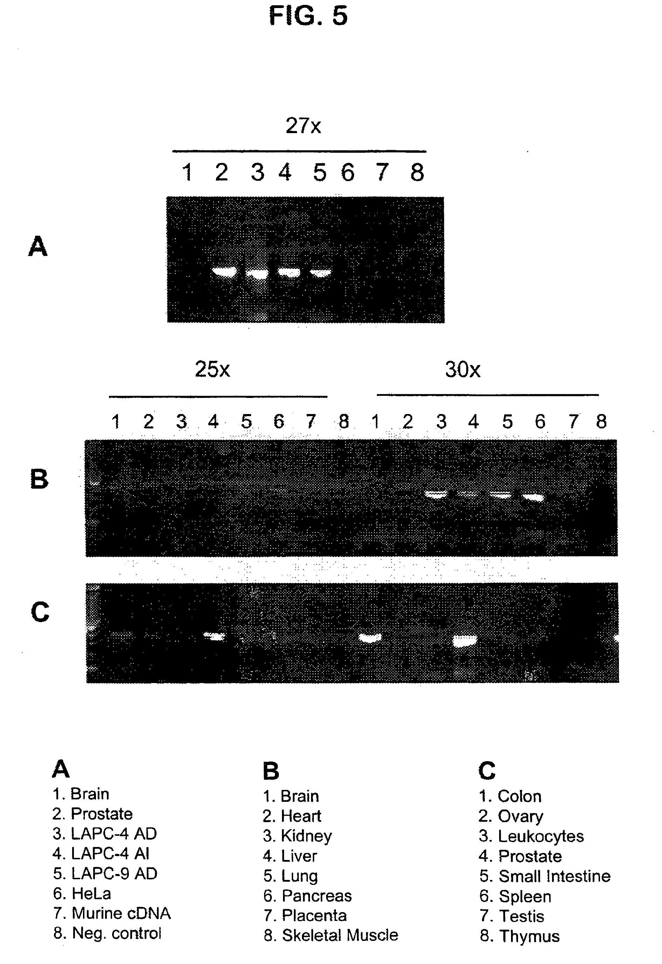

FIG. 5. RT-PCR analysis of 20P1F12/TMPRSS2 gene expression in prostate cancer xenografts, normal prostate, and other tissues and cell lines, showing approximately equal levels of expression in normal prostate and three prostate cancer xenografts (Panel A); and showing largely prostate specific expression in normal human tissues, with significantly lower expression levels detectable in colon, pancreas, kidney and lung (Panels B and C).

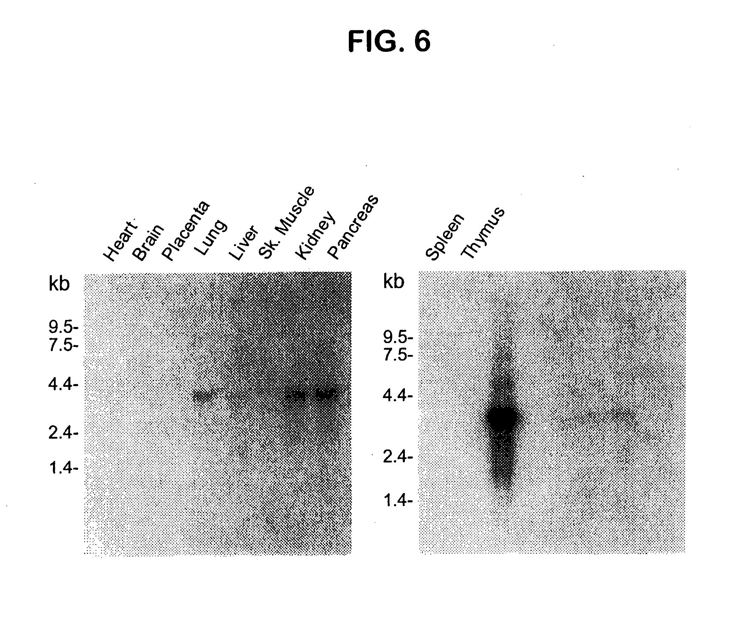

FIG. 6. Northern blot analysis of 20P1F12/TMPRSS2 gene expression in normal human tissues and prostate cancer xenografts using labeled clone 20P1F12 cDNA probe. Panels A and B: Expression in 16 normal tissues largely restricted to prostate; with kidney, pancreas and lung showing 10- to 20- fold lower expression levels. Panel C: Expression in LAPC-4 prostate cancer xenografts and various cell lines showing high level expression in prostate cancer xenografts, some of the prostate cancer cell lines, and in a colon carcinoma cell line. Except for LAPC-9 AI, expression of 20P1F12/TMPRSS2 in the xenografts was comparable to levels observed in normal prostate samples. In addition, lower level expression in the epidermoid carcinoma line A431 was observed.

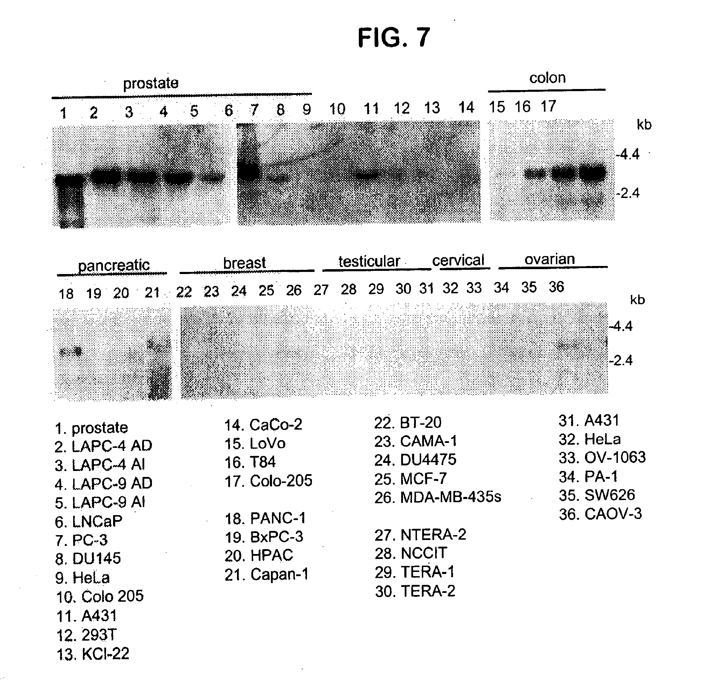

FIG. 7. Expression of 20P1F12/TMPRSS2 in prostate and colon cancer cell lines. Xenograft and cell line filters were prepared with 10 µg of total RNA per lane. The blots were analyzed using a 20P1F12/TMPRSS2 derived gene fragment probe. All RNA samples were normalized by ethidium bromide staining. Kilobases= kb.

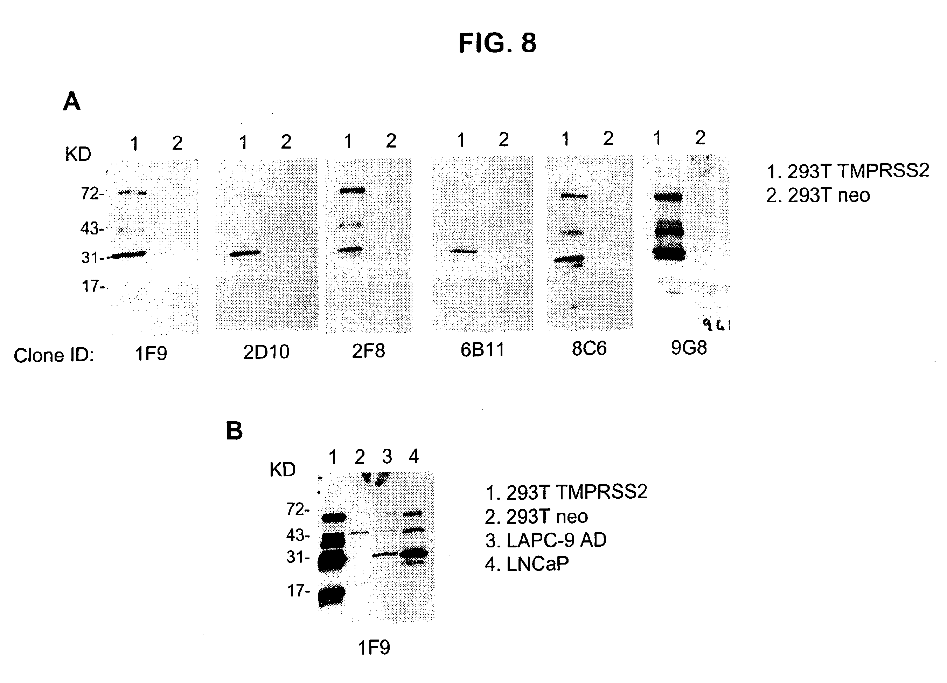

FIG. 8. Characterization of Monoclonal antibodies directed against 20P1F12/TMPRSS2. Monoclonal antibodies towards 20P1F12/TMPRSS2 were generated using a purified GST-20P1F12/TMPRSS2 fusion protein as described in Example 5. Hybridoma supernatants were initially screened by ELISA against purified 20P1F12/TMPRSS2 protein cleaved from the GST-fusion. A secondary screen involved western blotting against lysates derived from 293T cells transfected with a retroviral vector encoding 20P1F12/TMPRSS2. (A) Six mAbs (1 F9, 2D10, 2F8, 6B11, 8C6 and 9G8) that specifically recognize 20P1 F12/TMPRSS2 were used to probe western blots from cell lysates derived from 293T cells transfected with either 20P1F12/TMPRSS2 (lane 1) or neo (as a control, lane 2). (B) Cell lysates from 293T cells transfected with 20P1F12/TMPRSS2 (lane 1) or neo (as a control, lane 2), LAPC-9 AD and LNCaP were probed with 1 F9 anti-TMPRSS2 mAb. Molecular weight standards are indicated on the side in kilodaltons (KD).

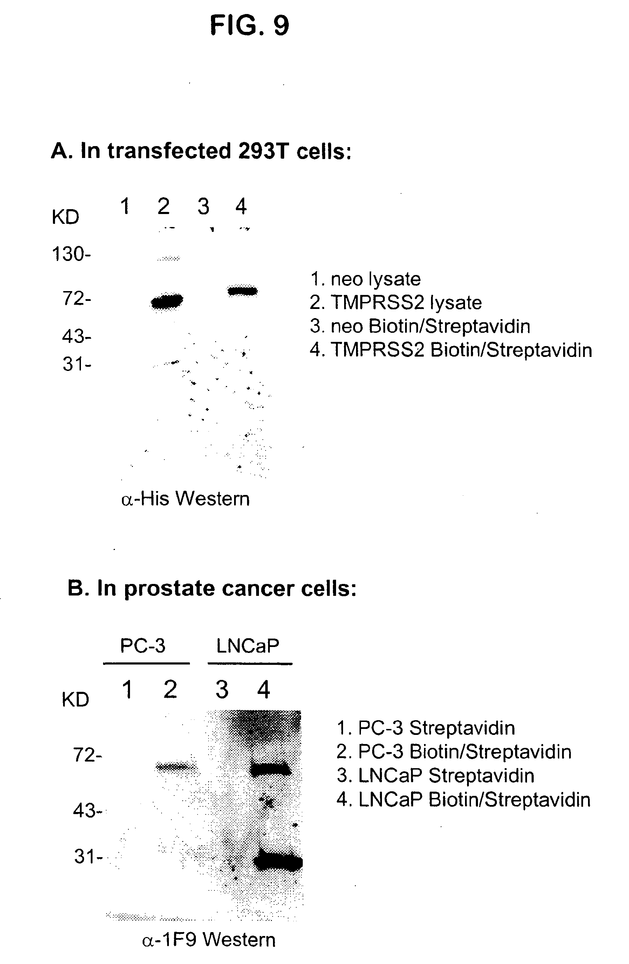

FIG. 9. Cell surface biotinylation of 20P1F12/TMPRSS2. (A) His-tagged 20P1 F12/TMPRSS2 or neo (as a control) were transfected into 293T cells. Intact cells were incubated with biotin to biotinylate all cell surface proteins. Cell lysates were either analyzed by western blotting directly (lanes 1 and 2, or they were incubated with streptavidin to affinity purify all labeled cell surface proteins). Streptavidin purified cell surface proteins were analyzed by western blotting using anti-His antibodies (lanes 3 and 4). Biotinylated protein was only detected in 20P1F12/TMPRSS2 transfected cells.(B) Biotinylated PC-3 (lane 2) and LNCaP (Lane 4), and unlabelled PC-3 (lane 1) and LNCaP (lane 3) were incubated with streptavidin gel and then analyzed by western blotting using 1F9 mAb. 20P1F12/TMPRSS2 was only detected in biotinylated samples. Molecular weight standards are indicated on the side in kilodaltons (KD).

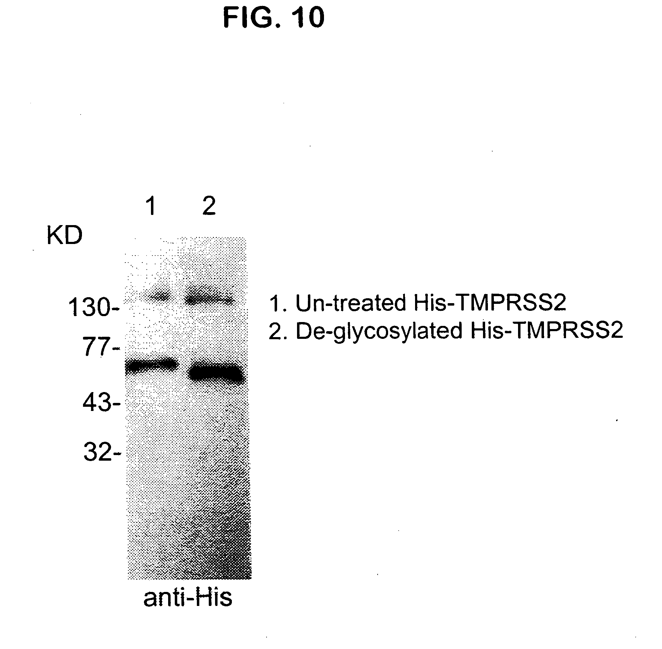

FIG. 10. De-glycosylation of 20P1F12/TMPRSS2 in transfected 293T cells. His-tagged 20P1F12/TMPRSS2 transfected into 293T cells was purified using Nickel-agarose. 20P1F12/TMPRSS2 protein was then de-glycosylated using N-glycosidase F. Untreated 20P1F12/TMPRSS2 (lane 1) and de-glycosylated protein (lane 2) were analyzed by western blotting using anti-His antibodies. A shift in molecular weight is detected with de-glycosylation. Molecular weight standards are indicated on the side in kilodaltons (KD).

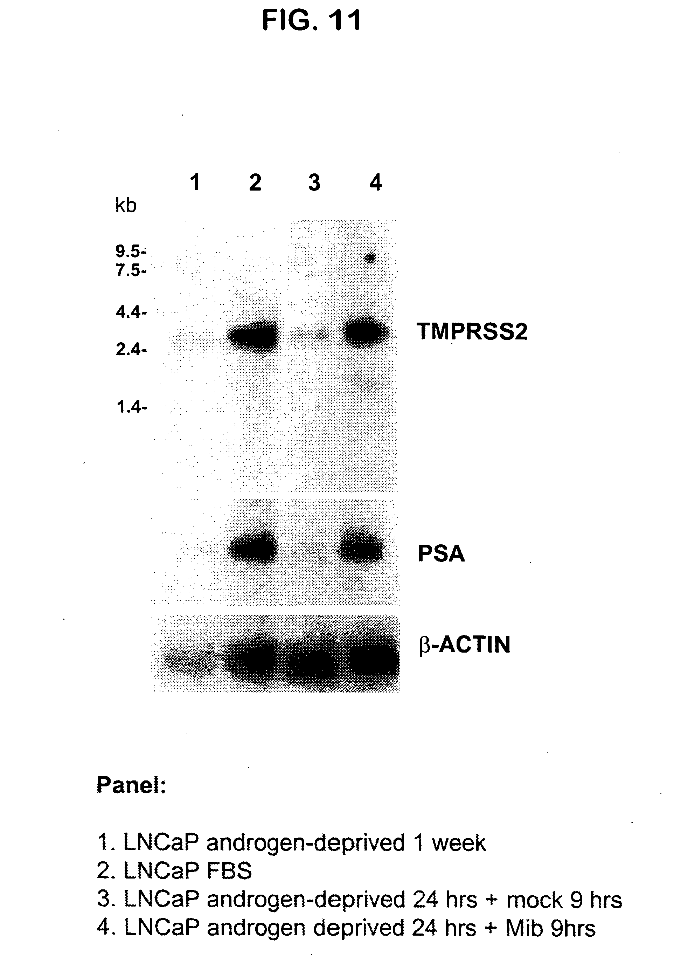

FIG. 11. Androgen regulation of 20P1F12/TMPRSS2 cell surface protease. LNCaP cells were deprived of androgen by growing cells in 2% charcoal-stripped fetal bovine serum for 1 week (lane 1), or 24 hours (lane 3). Androgen regulation was determined by stimulating 24 hour starved cells with 10 nM mibolerone (androgen analogue) for 9 hours (lane 4). Expression of 20P1F12/TMPRSS2 was compared to 20P1F12/TMPRSS2 levels in LNCAP cells growing in complete medium (lane 2) by northern blotting of 10 µg of RNA/lane probed with a 20P1F12/TMPRSS2 probe. Equal RNA loading was determined by ethidium bromide staining and subsequent probing with a β-acting probe. PSA levels were determined as a control for androgen regulation. Molecular weight standards are indicated on the side in kilobases (kb).

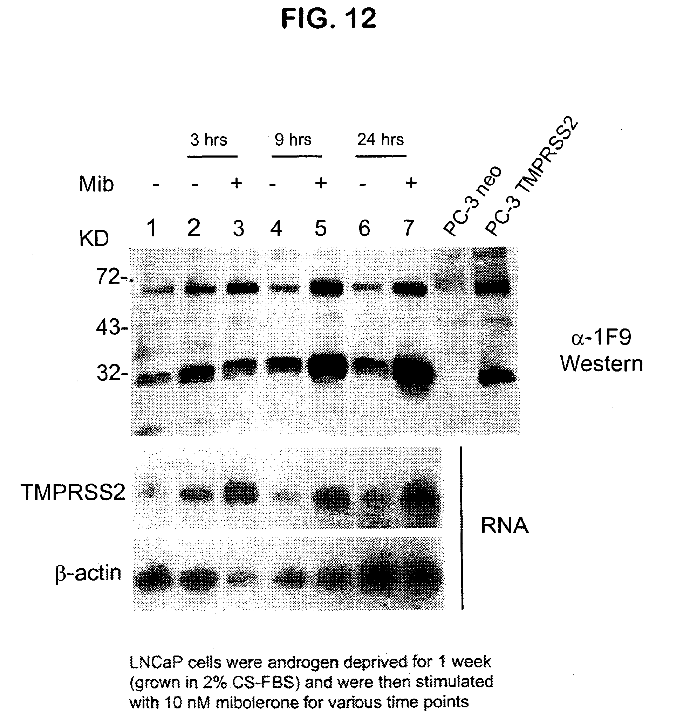

FIG. 12. Androgen regulation of 20P1F12/TMPRSS2 in LNCaP. LNCaP cells were deprived of androgen by growing cells in 2% charcoal-stripped fetal bovine serum for 1 week. Androgen regulation was determined by stimulating cells with mibolerone (Mib) for various time points. Expression of 20P1F12/TMPRSS2 was determined by western blotting of cell lysates using anti-1 F9 mAb. As additional controls cell lysates from PC-3 cells infected with either neo (as a control) or 20P1F12/TMPRSS2 were used. Equal protein loading was determined by probing the western blot with anti-Grb-2 antibodies (Transduction Laboratories)(data not shown). Protein expression of 20P1F12/TMPRSS2 was compared to RNA levels by northern blotting of 10 µg RNA/lane probed with a 20P1F12/TMPRSS2 probe. Equal RNA loading was determined by probing the northern blot with a β-acting probe.

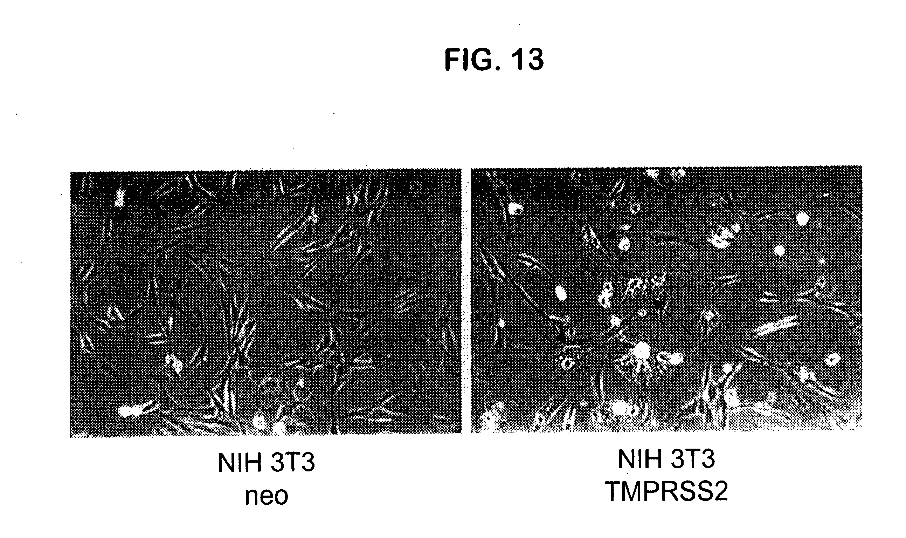

FIG. 13. Effect of 20P1F12/TMPRSS2 expression in NIH 3T3 cells. NIH 3T3 cells were infected with retrovirus encoding either neo (as a control) or 20P1F12/TMPRSS2. Forty-eight hours after infection the cells were analyzed by light microscopy. Cells that appeared to accumulate high numbers of vacuoles are indicated with arrows.

### DETAILED DESCRIPTION OF THE INVENTION

The present invention relates to methods and compositions for the diagnosis and therapy of prostate cancer which utilize isolated polynucleotides corresponding to the 20P1F12/TMPRSS2 gene, proteins encoded by the 20P1F12/TMPRSS2 gene and antibodies capable of specifically recognizing and binding to 20P1F12/TMPRSS2 proteins. The 20P1F12/TMPRSS2 gene encodes a predicted 492 amino acid multimeric protein containing a serine protease domain, a scavenger receptor cysteine-rich domain, an LDL receptor class A domain, and a predicted transmembrane domain as has been described for TMPRSS2 (Paoloni-Giacobino et al., 1997, Genomics 44: 309-320). Paoloni-Giacobino et al. found that the TMPRSS2 gene is expressed strongly in small intestine and only weakly in several other tissues and have also mapped the TMPRSS2 gene to chromosome 21. The physiological role of TMPRSS2 is unknown. Applicants have cloned a full length cDNA comprising the entire coding region of the 20P1F12/TMPRSS2 gene, but it contains several nucleotide sequence differences relative the published sequence of TMPRSS2. Five of these sequence differences result in amino acid differences. The nature and significance of these changes are presently unknown. In addition, applicants novel 20P1F12/TMPRSS2 has a completely different expression pattern in comparison to what has been known for the previously reported TMPRSS2.

Because 20P1F12/TMPRSS2 is a prostate specific protease, it is possible that it functions directly in the development and/or progression of prostate cancer, particularly in the development of metastatic disease. In this regard, proteases are known to be involved in invasion and metastasis of cancer cells (Henriet et al., 1999, APMIS 107(1):111-9; Rochefort et al., 1999, APMIS 107(1):86-95; Webber et al., 1995, Clin Cancer Res 1(10):1089-94; Duffy, 1996, Clin Cancer Res 2(4):613-8; Webber and Waghray, 1995 Clin Cancer Res 1(7):755-61). For instance, urokinase-type plasminogen activator (u-PA), cathepsin D and PSA are thought to contribute to the ability of prostate cancer cells to metastasize. The potential direct involvement of 20P1 F12/TMPRSS2 function in prostate cancer, and particularly in metastasis, may be evaluated as described in Example 5.

Interestingly, the 20P1F12/TMPRSS2 and TMPRSS2 primary structure contains protein-protein interaction domains and an extracellular protease domain. The function of 20P1F12/TMPRSS2 and TMPRSS2 is unclear. The function of 20P1F12/TMPRSS2 and TMPRSS2 may involve binding to substrate proteins in the extracellular milieu through its SRCR and/ or LDLA domains. Examples of proteins that exhibit SRCR domains include: CD6, an adhesion molecule that binds to ALCAM (activated leukocyte cell adhesion molecule) and mediates thymocyte-thymic epithelium cell binding (Whitney et al., 1995, J Biol Chem 270:18187); CD5 (Ly-1) a T-cell protein that binds CD72 on B-cells and may be involved in T-B cell communication (Luo et al., 1992, J Immunol 148:1630); BSSP-3, a brain-specific serine protease with a kringle-like structure and three scavenger receptor cysteine-rich motifs (Yamamura et al., 1997, Biochem Biophys Res Commun 239:386).

The protease domain of 20P1F12/TMPRSS2 is most homologous to the protease domain of hepsin (TMPRSS1), a transmembrane serine protease that is highly expressed in liver and up-regulated in ovarian cancer Leyus et al., 1988, Biochemistry 27: 1067-74; Tanimoto et al., 1997, Cancer Res. 57: 2884-2887).

The invention is based, in part, upon the isolation of a cDNA fragment corresponding to the 20P1 F12/TMPRSS2 gene by Suppression Subtraction Hybridization cloning and upon the detailed molecular and biochemical characterization studies described in the Examples. The initially isolated cDNA fragment, clone 20P1F12, showed identity in an overlapping part of the 3' untranslated sequence of the recently described full length cDNA encoding TMPRSS2. Primers designed to specifically amplify the gene corresponding to 20P1F12 were then used to characterize 20P1F12/TMPRSS2 expression in prostate cancer xenografts, normal prostate, and a variety of other normal tissues. A full length cDNA comprising the entire coding sequence of the 20P1F12/TMPRSS2 gene has been isolated and sequenced and is provided herein.

The nucleotide and deduced amino acid sequences of the novel 20P1F12/TMPRSS2 gene (also designated 20P1F12-GTC1 herein) are shown in FIG. 1. There are significant differences in the amino acid sequences encoded by the 20P1 F12-GTC1/TMPRSS2 gene compared to the previously reported sequence of TMPRSS2 (see amino acid alignment in FIG. 3). For example, four of the amino acid differences are in the protease domain, three of which are non-conservative amino acid differences and which could affect protease function and/or specificity. Applicants' novel 20P1F12/TMPRSS2 protein has been extensively characterized, as further described in the Examples sections herein. The 20P1F12/TMPRSS2 protein is a glycosylated type II transmembrane protein with an extracellular C-terminal protease domain. The 20P1F12/TMPRSS2 gene is androgen-regulated. The 20P1F12/TMPRSS2 protein is expressed on the cell surface. Expression of the 20P1 F12/TMPRSS2 gene in normal tissues is prostate-specific. Expression of 20P1 F12/TMPRSS2 is also observed in prostate cancer, including high level expression in advanced and metastatic disease. In addition, 20P1F12 appears to be over-expressed in colon cancer and may also be expressed in other cancers.

In addition to the differences in structure between applicants' the 20P1 F12-GTC1/TMPRSS2 gene (FIG. 1) and the previously reported sequence (FIG. 2), the results of applicants expression analysis are contrary to those reported by Paoloni-Giacobino et al. In particular, applicants analysis of 20P1F12/TMPRSS2 gene expression by RT-PCR in 16 normal tissues shows the highest level expression in prostate, with substantially lower levels detected in colon, pancreas, kidney, liver and lung and no detectable expression in small intestine (FIG. 5, Panels B and C). Similar results were obtained on Northern blot analysis, although the expression level detected in prostate by Northern blot is extremely high relative to these other tissues in which only very low level expression is detected (FIG. 6, Panels A and B).

Expression analysis also shows high level expression of 20P1F12/TMPRSS2 in all prostate cancer xenografts tested, at approximately the same levels seen in normal prostate (FIG. 5, Panel A). Northern blot analysis shows similar results, with somewhat lower level expression detected in the LAPC-9 xenograft relative to the LAPC-4 xenografts and normal prostate; expression is also detected in some of the prostate cancer cell lines analyzed (FIG. 6, Panel C). The 20P1F12/TMPRSS2 gene is also expressed in a number of prostate cancer cell lines (FIG. 7). These results indicate that the 20P1F12/TMPRSS2 gene is primarily a prostate specific gene which may be involved in the development and/or progression of prostate cancer. In addition, high level expression of 20P1F12/TMPRSS2 was detected by Northern blot in a number of colon carcinoma cell lines (FIG. 6, Panel C; FIG. 7). Expression of 20P1F12/TMPRSS2 in colon cancer may provide a molecular basis for detecting, diagnosing, prognosing and/or treating colon cancer.

Thus, the invention provides a unique and useful 20P1F12/TMPRSS2 gene (and protein), having the nucleotide and encoded amino acid sequences as shown in FIG. 1. Nucleotide probes corresponding to all or part of the 20P1F12/TMPRSS2 cDNA sequences disclosed herein (FIGS. 1 and 4) are provided and may be used to isolate or identify other cDNAs encoding all or part of the 20P1F12/TMPRSS2 gene sequence. The invention further provided primers capable of specifically amplifying the 20P1 F12/TMPRSS2 gene or its RNA transcripts. The invention further provides isolated polynucleotides containing coding sequences of the 20P1F12/TMPRSS2 gene product(s). Such polynucleotides may be used to express 20P1F12/TMPRSS2 encoded proteins and peptides having a number of further uses. 20P1F12/TMPRSS2 gene probes and primers may also be used to detect the presence or absence of 20P1F12/TMPRSS2 mRNA in various biological samples, for detecting prostate cancer cells and other cells expressing 20P1 F12/TMPRSS2, for generating tumor vaccines, and in molecular diagnostic and prognostic assays for prostate cancer. Polynucleotides corresponding or complementary to the 20P1F12/TMPRSS2 gene may be useful in methods for treating prostate cancer, such as, for example, in modulating or inhibiting 20P1 F12/TMPRSS2 biological activity.

More specifically, a 20P1F12/TMPRSS2 polynucleotide useful in the practice of the invention may comprise a polynucleotide having the nucleotide sequence of human 20P1F12/TMPRSS2 as shown in FIG. 1 (SEQ ID NO. XX) or the nucleotide sequence of the previously reported TMPRSS2 as shown in FIG. 2 (SEQ ID NO: XX), a sequence complementary to either of the foregoing, or a polynucleotide fragment of any of the foregoing. Another embodiment comprises a polynucelotide which encodes the 20P1F12/TMPRSS2 protein amino acid sequence as shown in FIG. 1 (SEQ ID NO. XX).

Included within the scope of this aspect of the invention are genomic DNA, cDNAs, ribozymes, and antisense molecules, as well as nucleic acid molecules based on an alternative backbone or including alternative bases, whether derived from natural sources or synthesized. For example, antisense molecules can be RNAs or other molecules, including peptide nucleic acids (PNAs) or non-nucleic acid molecules such as phosphorothioate derivatives, that specifically bind DNA or RNA in a base pair-dependent manner. A skilled artisan can readily obtain these classes of nucleic acid molecules using the 20P1F12/TMPRSS2 polynucleotides and polynucleotide sequences disclosed herein.

Further specific embodiments of this aspect of the invention include the use of primers and primer pairs, which allow the specific amplification of the polynucleotides of the invention or of any specific parts thereof, and probes that selectively or specifically hybridize to nucleic acid molecules of the invention or to any part thereof. Probes may be labeled with a detectable marker, such as, for example, a radioisotope, fluorescent compound, bioluminescent compound, a chemiluminescent compound, metal chelator or enzyme. Such probes and primers can be used to detect the presence of a 20P1F12/TMPRSS2 polynucleotide in a sample and as a means for detecting a cell expressing a 20P1F12/TMPRSS2 protein. An example of such a probe is a polypeptide comprising all or part of the human 20P1F12/TMPRSS2 cDNA sequence shown in FIG. 1 (SEQ ID NO. XX). Examples of primer pairs capable of specifically amplifying 20P1 F12/TMPRSS2 mRNAs are also described in the Examples which follow. As will be understood by the skilled artisan, a great many different primers and probes may be prepared based on the sequences provided in herein and used effectively to amplify and/or detect a 20P1F12/TMPRSS2 mRNA.

The 20P1F12/TMPRSS2 polynucleotides of the invention are useful for a variety of purposes, including but not limited to their use as probes and primers for the amplification and/or detection of the 20P1F12/TMPRSS2 gene, mRNA, or fragments thereof; as reagents for the diagnosis and/or prognosis of prostate and colon cancer; as coding sequences capable of directing the expression of 20P1F12/TMPRSS2 polypeptides; as tools for modulating or inhibiting the expression of the 20P1F12/TMPRSS2 gene and/or translation of the 20P1F12/TMPRSS2 transcript; and as therapeutic agents.

The invention also provides 20P1 F12/TMPRSS2 proteins and polypeptides which may be used, for example, to generate antibodies or for use as cancer vaccines. Antibodies capable of specifically binding to and identifying 20P1F12/TMPRSS2 proteins or polypeptides may be used to detect the expression of 20P1 F12/TMPRSS2, determine its subcellular location, detect and image prostate cancer cells and prostate tumors, and modulate or inhibit 20P1F12/TMPRSS2 biological activity. Antibodies may also used therapeutically as described further below. Methods for the generation of polyclonal and monoclonal antibodies are well known in the art.

The invention also provides recombinant DNA or RNA molecules containing a 20P1F12/TMPRSS2 polynucleotide, including but not limited to phages, plasmids, phagemids, cosmids, YACs, BACs, as well as various viral and non-viral vectors well known in the art, and cells transformed or transfected with such recombinant DNA or RNA molecules. As used herein, a recombinant DNA or RNA molecule is a DNA or RNA molecule that has been subjected to molecular manipulation in vitro. Methods for generating such molecules are well known (see, for example, Sambrook et al, 1989, supra).

The invention further provides a host-vector system comprising a recombinant DNA molecule containing a 20P1F12/TMPRSS2 polynucleotide within a suitable prokaryotic or eukaryotic host cell. Examples of suitable eukaryotic host cells include a yeast cell, a plant cell, or an animal cell, such as a mammalian cell or an insect cell (e.g., a baculovirus-infectible cell such as an Sf9 cell). Examples of suitable mammalian cells include various prostate cancer cell lines such LnCaP, PC-3, DU145, LAPC-4, TsuPr1, other transfectable or transducible prostate cancer cell lines, as well as a number of mammalian cells routinely used for the expression of recombinant proteins (e.g., COS, CHO, 293, 293T cells). More particularly, a polynucleotide comprising the coding sequence of a 20P1 F12/TMPRSS2 may be used to generate 20P1F12/TMPRSS2 proteins or fragments thereof using any number of host-vector systems routinely used and widely known in the art.

A wide range of host-vector systems suitable for the expression of 20P1F12/TMPRSS2 proteins or fragments thereof are available, see for example, Sambrook et al., 1989, supra; Current Protocols in Molecular Biology, 1995, supra). Preferred vectors for mammalian expression include but are not limited to pcDNA 3.1 myc-His-tag (Invitrogen) and the retroviral vector pSRαtkneo (Muller et al., 1991, MCB 11:1785). Using these expression vectors, 20P1F12/TMPRSS2 may be preferably expressed in several prostate and non-prostate cancer cell lines, including for example 3T3, 293, 293TPC-3, LNCaP and TsuPr1. The host-vector systems of the invention are useful for the production of a 20P1 F12/TMPRSS2 protein or fragment thereof. Such host-vector systems may be employed to study the functional properties of 20P1F12/TMPRSS2 and 20P1 F12/TMPRSS2 mutations.

Proteins encoded by the 20P1F12/TMPRSS2 genes, or by fragments thereof, will have a variety of uses, including but not limited to generating antibodies and in methods for identifying ligands and other agents and cellular constituents that bind to a 20P1F12/TMPRSS2 gene product. Such proteins may also be used as cancer vaccines. Antibodies raised against a 20P1F12/TMPRSS2 protein or fragment thereof may be useful in diagnostic and prognostic assays, imaging methodologies (including, particularly, cancer imaging), and therapeutic methods in the management of human cancers characterized by expression of a 20P1F12/TMPRSS2 protein, such as prostate and colon cancers. Various immunological assays useful for the detection of 20P1 F12/TMPRSS2 proteins are contemplated, including but not limited to various types of radioimmunoassays, enzyme-linked immunosorbent assays (ELISA), enzyme-linked immunofluorescent assays (ELIFA), immunocytochemical methods, and the like. Such antibodies may be labeled and used as immunological imaging reagents capable of detecting prostate cells (e.g., in radioscintigraphic imaging methods).

In a specific embodiment, a novel 20P1F12/TMPRSS2 protein having the amino acid sequence of human 20P1F12/TMPRSS2 is provided in FIG. 1 (SEQ ID NO. XX). Fusion proteins which combine all or part of 20P1F12/TMPRSS2 with a heterologous polypeptide are also contemplated. The 20P1F12/TMPRSS2 protein of the invention may be embodied in many forms, preferably in isolated form. As used herein, the protein is said to be "isolated" when physical, mechanical or chemical methods are employed to remove the 20P1 F12/TMPRSS2 protein from cellular constituents that are normally associated with the protein. A skilled artisan can readily employ standard purification methods to obtain an isolated 20P1F12/TMPRSS2 protein. A purified 20P1F12/TMPRSS2 protein molecule will be substantially free of other proteins or molecules which impair the binding of 20P1 F12/TMPRSS2 to antibody or other ligand. The nature and degree of isolation and purification will depend on the intended use. Embodiments of a 20P1F12/TMPRSS2 protein include a purified 20P1F12/TMPRSS2 protein and a functional, soluble 20P1F12/TMPRSS2 protein. In one form, such functional, soluble 20P1F12/TMPRSS2 proteins or fragments thereof retain the ability to bind antibody or other ligand.

Recombinant methods can be used to generate nucleic acid molecules that encode the 20P1F12/TMPRSS2 protein. In this regard, the 20P1F12/TMPRSS2-encoding nucleic acid molecules described herein provide means for generating defined fragments of the 20P1F12/TMPRSS2 protein. Such 20P1F12/TMPRSS2 polypeptides are particularly useful in generating domain specific antibodies (e.g., antibodies recognizing an extracellular epitope of the 20P1F12/TMPRSS2 protein), identifying agents or cellular factors that bind to a particular 20P1F12/TMPRSS2 domain, and in various therapeutic contexts, including but not limited to cancer vaccines. 20P1F12/TMPRSS2 polypeptides containing particularly interesting structures can be predicted and/or identified using various analytical techniques well known in the art, including, for example, the methods of Chou-Fasman, Garnier-Robson, Kyte-Doolittle, Eisenberg, Karplus-Schultz or Jameson-Wolf analysis, or on the basis of immunogenicity.

Also described herein are antibodies that immunospecifically bind to the 20P1F12/TMPRSS2 protein and polypeptide fragments thereof. The most preferred antibodies will selectively bind to a 20P1F12/TMPRSS2 protein and will not bind (or will bind weakly) to non-20P1F12/TMPRSS2 proteins and polypeptides. Anti-20P1F12/TMPRSS2 antibodies that are particularly contemplated include monoclonal and polyclonal antibodies as well as fragments containing the antigen binding domain and/or one or more complementarity determining regions of these antibodies. As used herein, an antibody fragment is defined as at least a portion of the variable region of the immunoglobulin molecule which binds to its target, i.e., the antigen binding region.

For some applications, it may be desirable to generate antibodies which specifically react with a particular 20P1F12/TMPRSS2 protein and/or an epitope within a particular structural domain. For example, preferred antibodies useful for cancer diagnostic imaging purposes are those with react with an epitope in an extracellular region of the 20P1F12/TMPRSS2 protein as expressed in cancer cells. Such antibodies may be generated by using the 20P1F12/TMPRSS2 protein, or using peptides derived from predicted extracellular or other domains of 20P1F12/TMPRSS2, and used as an immunogen.

The 20P1F12/TMPRSS2 antibodies described herein may be particularly useful in prostate and colon cancer diagnostic and prognostic assays, imaging methodologies, and therapeutic strategies. The invention provides various immunological assays useful for the detection and quantification of 20P1F12/TMPRSS2. Such assays generally comprise one or more 20P1F12/TMPRSS2 antibodies capable of recognizing and binding a 20P1F12/TMPRSS2, and may be performed within various immunological assay formats well known in the art, including but not limited to various types of radioimmunoassays, enzyme-linked immunosorbent assays (ELISA), enzyme-linked immunofluorescent assays (ELIFA), and the like. In addition, immunological imaging methods capable of detecting prostate cancer are also provided by the invention, including but limited to radioscintigraphic imaging methods using labeled 20P1F12/TMPRSS2 antibodies. Such assays may be clinically useful in the detection, monitoring, and prognosis of prostate cancer, particularly advanced prostate cancer.

20P1F12/TMPRSS2 antibodies may also be used in methods for purifying 20P1F12/TMPRSS2 proteins and polypeptides and for isolating 20P1F12/TMPRSS2 homologues and related molecules. For example, in one embodiment, the method of purifying a 20P1F12/TMPRSS2 protein comprises incubating a 20P1F12/TMPRSS2 antibody, which has been coupled to a solid matrix, with a lysate or other solution containing 20P1F12/TMPRSS2 under conditions which permit the 20P1F12/TMPRSS2 antibody to bind to 20P1F12/TMPRSS2; washing the solid matrix to eliminate impurities; and eluting the 20P1F12/TMPRSS2 from the coupled antibody. Other uses of the 20P1F12/TMPRSS2 antibodies described herein include generating anti-idiotypic antibodies that mimic the 20P1F12/TMPRSS2 protein.

20P1F12/TMPRSS2 antibodies may also be used therapeutically by, for example, modulating or inhibiting the biological activity of a 20P1F12/TMPRSS2 protein or targeting and destroying prostate cancer cells expressing a 20P1F12/TMPRSS2 protein. Antibody therapy of prostate and colon cancer is described in further detail below.

Various methods for the preparation of antibodies are well known in the art. For example, antibodies may be prepared by immunizing a suitable mammalian host using a 20P1F12/TMPRSS2 protein, peptide, or fragment, in isolated or immunoconjugated form (Antibodies: A Laboratory Manual, CSH Press, Eds., Harlow, and Lane (1988); Harlow, Antibodies, Cold Spring Harbor Press, NY (1989)). In addition, fusion proteins of 20P1F12/TMPRSS2 may also be used, such as a 20P1F12/TMPRSS2 GST-fusion protein. In a particular embodiment, a GST fusion protein comprising all or most of the open reading frame amino acid sequence of FIG. 1 may be produced and used as an immunogen to generate appropriate antibodies. As described in Example 5, such a GST fusion was used to generate several monoclonal antibodies which immunospecifically react with 20P1F12/TMPRSS2. Cells expressing or overexpressing 20P1F12/TMPRSS2 may also be used for immunizations. Similarly, any cell engineered to express 20P1F12/TMPRSS2 may be used. This strategy may result in the production of monoclonal antibodies with enhanced capacities for recognizing endogenous 20P1F12/TMPRSS2. Additional strategies for generating 20P1F12/TMPRSS2 antibodies are described in Example 5 herein.

The amino acid sequence of 20P1F12/TMPRSS2 as shown in FIG. 1 (SEQ ID NO. XX) may be used to select specific regions of the 20P1F12/TMPRSS2 protein for generating antibodies. For example, hydrophobicity and hydrophilicity analyses of the 20P1 F12/TMPRSS2 amino acid sequence may be used to identify hydrophilic regions in the 20P1F12/TMPRSS2 structure. Regions of the 20P1F12/TMPRSS2 protein that show immunogenic structure, as well as other regions and domains, can readily be identified using various other methods known in the art, such as Chou-Fasman, Garnier-Robson, Kyte-Doolittle, Eisenberg, Karplus-Schultz or Jameson-Wolf analysis.

Methods for preparing a protein or polypeptide for use as an immunogen and for preparing immunogenic conjugates of a protein with a carrier such as BSA, KLH, or other carrier proteins are well known in the art. In some circumstances, direct conjugation using, for example, carbodiimide reagents may be used; in other instances linking reagents such as those supplied by Pierce Chemical Co., Rockford, IL, may be effective. Administration of a 20P1F12/TMPRSS2 immunogen is conducted generally by injection over a suitable time period and with use of a suitable adjuvant, as is generally understood in the art. During the immunization schedule, titers of antibodies can be taken to determine adequacy of antibody formation.

Anti-20P1F12/TMPRSS2 monoclonal antibodies are preferred and may be produced by various means well known in the art. For example, immortalized cell lines which secrete a desired monoclonal antibody may be prepared using the standard method of Kohler and Milstein or modifications which effect immortalization of lymphocytes or spleen cells, as is generally known. The immortalized cell lines secreting the desired antibodies are screened by immunoassay in which the antigen is the 20P1F12/TMPRSS2 protein or 20P1F12/TMPRSS2 fragment. When the appropriate immortalized cell culture secreting the desired antibody is identified, the cells may be expanded and antibodies produced either from in vitro cultures or from ascites fluid.

The antibodies or fragments may also be produced, using current technology, by recombinant means. Regions that bind specifically to the desired regions of the 20P1F12/TMPRSS2 protein can also be produced in the context of chimeric or CDR grafted antibodies of multiple species origin. Humanized or human 20P1F12/TMPRSS2 antibodies may also be produced and are preferred. Various approaches for producing such humanized antibodies are known, and include chimeric and CDR grafting methods; methods for producing fully human monoclonal antibodies include phage display and transgenic methods (for review, see Vaughan et al., 1998, Nature Biotechnology 16: 535-539).

Fully human 20P1F12/TMPRSS2 monoclonal antibodies may be generated using cloning technologies employing large human Ig gene combinatoerial libraries (i.e., phage display)(Griffiths and Hoogenboom, Building an in vitro immune system: human antibodies from phage display libraries. In: Protein Engineering of Antibody Molecules for Prophylactic and Therapeutic Applications in Man. Clark, M. (Ed.), Nottingham Academic, pp 45-64 (1993); Burton and Barbas, Human Antibodies from combinatorial libraries. Id., pp 65-82). Fully human 20P1F12/TMPRSS2 monoclonal antibodies may also be produced using transgenic mice engineered to contain human immunoglobulin gene loci as described in PCT Patent Application WO98/24893, Jakobovits et al., published December 3, 1997 (see also, Kucherlapati and Jakobovits, 1998, Exp. Opin. Invest. Drugs 7(4): 607-614). This method avoids the in vitro manipulation required with phage display technology and efficiently produces high affinity authentic human antibodies.

Reactivity of 20P1F12/TMPRSS2 antibodies with a 20P1F12/TMPRSS2 protein may be established by a number of well known means, including Western blot, immunoprecipitation, ELISA, and FACS analyses using, as appropriate, 20P1F12/TMPRSS2 proteins, peptides, 20P1F12/TMPRSS2-expressing cells or extracts thereof.

A 20P1F12/TMPRSS2 antibody or fragment thereof described herein may be labeled with a detectable marker or conjugated to a second molecule, such as a cytotoxic or therapeutic agent, and used for targeting a 20P1F12/TMPRSS2 positive cell (Vitetta, E.S. et al., 1993, Immunotoxin therapy, in DeVita, Jr., V.T. et al., eds, Cancer: Principles and Practice of Oncology, 4th ed., J.B. Lippincott Co., Philadelphia, 2624-2636). Suitable detectable markers include, but are not limited to, a radioisotope, a fluorescent compound, a bioluminescent compound, chemiluminescent compound, a metal chelator or an enzyme.

The 20P1F12/TMPRSS2 protein is a cell surface serine protease that may be involved in invasion and metastasis of prostate and colon cancer. Accordingly, 20P1F12/TMPRSS2 may be ideal target for therapeutic intervention. Its extracellular protease domain may be a potential drug target, while the whole extracellular domain may be a potential therapeutic antibody target. Therefore, the invention provides various immunotherapeutic compositions and methods for treating prostate and colon cancer, including antibody therapy, in vivo vaccines, and ex vivo immunotherapy approaches, which utilize polynucleotides and polypeptides corresponding to 20P1F12/TMPRSS2 and anti-20P1F12//TMPRSS2 antibodies.

In one approach, anti-20P1F12/TMPRSS2 antibodies may be used to treat prostate cancer. For example, unconjugated anti-20P1F12/TMPRSS2 antibody may be introduced into a patient such that the antibody binds to 20P1F12/TMPRSS2 on prostate cancer cells and mediates the destruction of the cells and the tumor. The therapeutic mechanism of action may include complement-mediated cytolysis, antibody-dependent cellular cytotoxicity, altering the physiologic function of 20P1F12/TMPRSS2, and/or the inhibition of ligand binding or signal transduction pathways. Anti-20P1F12/TMPRSS2 antibodies conjugated to toxic agents such as ricin, or to therapeutic agents, may also be used therapeutically to deliver the toxic or therapeutic agent directly to 20P1F12/TMPRSS2-bearing prostate tumor cells and thereby destroy the tumor.

Prostate cancer immunotherapy using anti-20P1F12/TMPRSS2 antibodies may follow the teachings generated from various approaches which have been successfully employed with respect to other types of cancer, including but not limited to colon cancer (Arlen et al., 1998, Crit Rev Immunol 18: 133-138), multiple myeloma (Ozaki et al., 1997, Blood 90: 3179-3186; Tsunenari et al., 1997, Blood 90: 2437-2444), gastric cancer (Kasprzyk et al., 1992, Cancer Res 52: 2771-2776), B-cell lymphoma (Funakoshi et al., 1996, J Immunther Emphasis Tumor Immunol 19: 93-101), leukemia (Zhong et al., 1996, Leuk Res 20: 581-589), colorectal cancer (Moun et al., 1994, Cancer Res 54: 6160-6166); Velders et al., 1995, Cancer Res 55: 4398-4403), and breast cancer (Shepard et al., 1991, J Clin Immunol 11:117-127).

20P1F12/TMPRSS2 antibodies may be introduced into a patient such that the antibody binds to 20P1F12/TMPRSS2 on the cancer cells and mediates the destruction of the cells and the tumor and/or inhibits the growth of the cells or the tumor. Mechanisms by which such antibodies exert a therapeutic effect may include complement-mediated cytolysis, antibody-dependent cellular cytotoxicity, modulating the physiologic function of 20P1F12/TMPRSS2, inhibiting ligand binding or signal transduction pathways, modulating tumor cell differentiation, altering tumor angiogenesis factor profiles, and/or by inducing apoptosis. 20P1 F12/TMPRSS2 antibodies conjugated to toxic or therapeutic agents may also be used therapeutically to deliver the toxic or therapeutic agent directly to 20P1F12/TMPRSS2-bearing tumor cells.

Although 20P1F12/TMPRSS2 antibody therapy may be useful for all stages of the foregoing cancers, antibody therapy may be particularly appropriate in advanced or metastatic prostate cancers. In particular, because the 20P1F12/TMPRSS2 gene appears not to be regulated by androgen, anti-20P1F12/TMPRSS2 antibody therapy may be used to treat patients undergoing androgen ablation therapy. Combining the antibody therapy method of the invention with a chemotherapeutic regimen may be preferred in patients who have not received chemotherapeutic treatment, whereas treatment with the antibody therapy of the invention may be indicated for patients who have received one or more chemotherapy. Additionally, antibody therapy may also enable the use of reduced dosages of concomitant chemotherapy, particularly in patients that do not tolerate the toxicity of the chemotherapeutic agent very well.

It may be desirable for patients to be evaluated for the presence and level of 20P1F12/TMPRSS2, preferably using immunohistochemical assessments of tumor tissue, quantitative 20P1F12/TMPRSS2 imaging, or other techniques capable of reliably indicating the presence and degree of expression. Immunohistochemical analysis of tumor biopsies or surgical specimens may be preferred for this purpose. Methods for immunohistochemical analysis of tumor tissues are well known in the art.

Anti-20P1F12/TMPRSS2 monoclonal antibodies useful in treating prostate cancers include those which are capable of initiating a potent immune response against the tumor and those which are capable of direct cytotoxicity. In this regard, anti-20P1F12/TMPRSS2 mAbs may elicit tumor cell lysis by either complement-mediated or antibody-dependent cell cytotoxicity (ADCC) mechanisms, both of which require an intact Fc portion of the immunoglobulin molecule for interaction with effector cell Fc receptor sites or complement proteins. In addition, anti-20P1F12/TMPRSS2 mAbs which exert a direct biological effect on tumor growth are useful in the practice of the invention. Potential mechanisms by which such directly cytotoxic mAbs may act include inhibition of cell growth, modulation of cellular differentiation, modulation of tumor angiogenesis factor profiles, and the induction of apoptosis. The mechanism by which a particular anti-20P1F12/TMPRSS2 mAb exerts an anti-tumor effect may be evaluated using any number of in vitro assays designed to determine ADCC and complement-mediated cell lysis, as well as growth inhibition, modulation of apoptosis and inhibition of differentiation, and/or inhibition of angiogenesis, as is generally known in the art.

The anti-tumor activity of a particular anti-20P1F12/TMPRSS2 mAb, or combination of anti-20P1F12/TMPRSS2 mAbs, may be evaluated in vivo using a suitable animal model. For example, xenogenic prostate cancer models wherein human prostate cancer explants or passaged xenograft tissues are introduced into immune compromised animals, such as nude or SCID mice, are appropriate in relation to prostate cancer and have been described (Klein et al., 1997, Nature Medicine 3: 402-408). For Example, PCT Patent Application WO98/16628, Sawyers et al., published April 23, 1998, describes various xenograft models of human prostate cancer capable of recapitulating the development of primary tumors, micrometastasis, and the formation of osteoblastic metastases characteristic of late stage disease. Efficacy may be predicted using inhibition of tumor formation, tumor regression, metastasis, and the like.

It should be noted that the use of murine or other non-human monoclonal antibodies, human/mouse chimeric mAbs may induce moderate to strong immune responses in some patients. In the most severe cases, such an immune response may lead to the extensive formation of immune complexes which, potentially, can cause renal failure. Accordingly, preferred monoclonal antibodies used in the practice of the therapeutic methods of the invention are those which are either fully human or humanized and which bind specifically to the target 20P1F12/TMPRSS2 antigen with high affinity but exhibit low or no antigenicity in the patient.

The method of the invention contemplate the administration of single anti-20P1F12/TMPRSS2 mAbs as well as combinations, or "cocktails, of different mAbs. Such mAb cocktails may have certain advantages inasmuch as they contain mAbs which exploit different epitope specificity, different effector mechanisms, or combine directly cytotoxic mAbs with mAbs that rely on immune effector functionality. Such mAbs in combination may exhibit synergistic therapeutic effects. In addition, the administration of anti-20P1F12/TMPRSS2 mAbs may be combined with other therapeutic agents or radiation therapy, including but not limited to various chemotherapeutic agents, androgen-blockers, and immune modulators (e.g., IL-2, GM-CSF). The anti-20P1 F12/TMPRSS2 mAbs may be administered in their "naked" or unconjugated form, or may have therapeutic or toxic agents conjugated to them.

The anti-20P1F12/TMPRSS2 monoclonal antibodies used in the practice of the method of the invention may be formulated into pharmaceutical compositions comprising a carrier suitable for the desired delivery method. Suitable carriers include any material which when combined with the anti-20P1F12/TMPRSS2 mAbs retains the specificity and anti-tumor function of the antibody and is non-reactive with the subject's immune systems. Examples include, but are not limited to, any of a number of standard pharmaceutical carriers such as sterile phosphate buffered saline solutions, bacteriostatic water, and the like.

The anti-20P1F12/TMPRSS2 antibody formulations may be administered via any route capable of delivering the antibodies to the tumor site. Potentially effective routes of administration include, but are not limited to, intravenous, intraperitoneal, intramuscular, intratumor, intradermal, and the like. The preferred route of administration is by intravenous injection. A preferred formulation for intravenous injection comprises the anti-20P1F12/TMPRSS2 mAbs in a solution of preserved bacteriostatic water, sterile unpreserved water, and/or diluted in polyvinylchloride or polyethylene bags containing 0.9% sterile Sodium Chloride for Injection, USP. The anti-20P1F12/TMPRSS2 mAb preparation may be lyophilized and stored as a sterile powder, preferably under vacuum, and then reconstituted in bacteriostatic water containing, for example, benzyl alcohol preservative, or in sterile water prior to injection.

Treatment will generally involve the repeated administration of the anti-20P1F12/TMPRSS2 antibody preparation via an acceptable route of administration such as intravenous injection (IV), typically at a dose in the range of about 0.1 to about 10 mg/kg body weight. Doses in the range of 10-500 mg mAb per week may be effective and well tolerated. Based on clinical experience with the Herceptin mAb in the treatment of metastatic breast cancer, an initial loading dose of approximately 4 mg/kg patient body weight IV followed by weekly doses of about 2 mg/kg IV of the anti-20P1F12/TMPRSS2 mAb preparation may represent an acceptable dosing regimen. Preferably, the initial loading dose is administered as a 90 minute or longer infusion. The periodic maintenance dose may be administered as a 30 minute or longer infusion, provided the initial dose was well tolerated. However, as one of skill in the art will understand, various factors will influence the ideal dose regimen in a particular case. Such factors may include, for example, the binding affinity and half life of the mAb or mAbs used, the degree of 20P1F12/TMPRSS2 overexpression in the patient, the extent of circulating shed 20P1F12/TMPRSS2 antigen, the desired steady-state antibody concentration level, frequency of treatment, and the influence of chemotherapeutic agents used in combination with the treatment method of the invention.

Optimally, patients should be evaluated for the level of circulating shed 20P1F12/TMPRSS2 antigen in serum in order to assist in the determination of the most effective dosing regimen and related factors. Such evaluations may also be used for monitoring purposes throughout therapy, and may be useful to gauge therapeutic success in combination with evaluating other parameters (such as serum PSA levels in prostate cancer therapy).

We also describe prostate cancer vaccines comprising a 20P1F12/TMPRSS2 protein or fragment thereof. The use of a tumor antigen in a vaccine for generating humoral and cell-mediated immunity for use in anti-cancer therapy is well known in the art and has been employed in prostate cancer using human PSMA and rodent PAP immunogens (Hodge et al., 1995, Int. J. Cancer 63: 231-237; Fong et al., 1997, J. Immunol. 159: 3113-3117). Such methods can be readily practiced by employing a 20P1F12/TMPRSS2 protein, or fragment thereof, or a 20P1 F12/TMPRSS2 - encoding nucleic acid molecule and recombinant vectors capable of expressing and appropriately presenting the 20P1F12/TMPRSS2 immunogen.

For example, viral gene delivery systems may be used to deliver a 20P1F12/TMPRSS2 - encoding nucleic acid molecule. Various viral gene delivery systems which can be used in the practice of this aspect of the invention include, but are not limited to, vaccinia, fowlpox, canarypox, adenovirus, influenza, poliovirus, adeno-associated virus, lentivirus, and sindbus virus (Restifo, 1996, Curr. Opin. Immunol. 8: 658-663). Non-viral delivery systems may also be employed by using naked DNA encoding a 20P1F12/TMPRSS2 protein or fragment thereof introduced into the patient (e.g., intramuscularly) to induce an anti-tumor response. In one embodiment, the full-length human 20P1F12/TMPRSS2 cDNA may be employed. In another embodiment, 20P1F12/TMPRSS2 nucleic acid molecules encoding specific cytotoxic T lymphocyte (CTL) epitopes may be employed. CTL epitopes can be determined using specific algorithms (e.g., Epimer, Brown University) to identify peptides within a 20P1F12/TMPRSS2 protein which are capable of optimally binding to specified HLA alleles.

Various ex vivo strategies may also be employed. One approach involves the use of dendritic cells to present 20P1F12/TMPRSS2 antigen to a patient's immune system. Dendritic cells express MHC class I and II, B7 costimulator, and IL-12, and are thus highly specialized antigen presenting cells. In prostate cancer, autologous dendritic cells pulsed with peptides of the prostate-specific membrane antigen (PSMA) are being used in a Phase I clinical trial to stimulate prostate cancer patients' immune systems (Tjoa et al., 1996, Prostate 28: 65-69; Murphy et al., 1996, Prostate 29: 371-380). Dendritic cells can be used to present 20P1F12/TMPRSS2 peptides to T cells in the context of MHC class I and II molecules. In one embodiment, autologous dendritic cells are pulsed with 20P1F12/TMPRSS2 peptides capable of binding to MHC molecules. In another embodiment, dendritic cells are pulsed with the complete 20P1F12/TMPRSS2 protein. Yet another embodiment involves engineering the overexpression of the 20P1F12/TMPRSS2 gene in dendritic cells using various implementing vectors known in the art, such as adenovirus (Arthur et al., 1997, Cancer Gene Ther. 4: 17-25), retrovirus (Henderson et al., 1996, Cancer Res. 56: 3763-3770), lentivirus, adeno-associated virus, DNA transfection (Ribas et al., 1997, Cancer Res. 57: 2865-2869), and tumor-derived RNA transfection (Ashley et al., 1997, J. Exp. Med. 186: 1177-1182).

Anti-idiotypic anti-20P1F12/TMPRSS2 antibodies can also be used in anti-cancer therapy as a vaccine for inducing an immune response to cells expressing a 20P1F12/TMPRSS2 protein. Specifically, the generation of anti-idiotypic antibodies is well known in the art and can readily be adapted to generate anti-idiotypic anti-20P1 F12/TMPRSS2 antibodies that mimic an epitope on a 20P1F12/TMPRSS2 protein (see, for example, Wagner et al., 1997, Hybridoma 16: 33-40; Foon et al., 1995, J Clin Invest 96: 334-342; Herlyn et al., 1996, Cancer Immunol Immunother 43: 65-76). Such an anti-idiotypic antibody can be used in anti-idiotypic therapy as presently practiced with other anti-idiotypic antibodies directed against tumor antigens.

Genetic immunization methods may be employed to generate prophylactic or therapeutic humoral and cellular immune responses directed against cancer cells expressing 20P1F12/TMPRSS2, particularly colon and prostate cancer cells. Constructs comprising DNA encoding a 20P1F12/TMPRSS2 protein/immunogen and appropriate regulatory sequences may be injected directly into muscle or skin of an individual, such that the cells of the muscle or skin take-up the construct and express the encoded 20P1F12/TMPRSS2 protein/immunogen. The 20P1F12/TMPRSS2 protein/immunogen may be expressed as a cell surface protein or be secreted. Expression of the 20P1F12/TMPRSS2 protein/immunogen results in the generation of prophylactic or therapeutic humoral and cellular immunity against prostate cancer. Various prophylactic and therapeutic genetic immunization techniques known in the art may be used (for review, see information and references published at internet address www.genweb.com).

Another aspect of the invention is directed to molecular diagnostic and diagnostic imaging methods which utilize the 20P1F12/TMPRSS2 polynucleotides and antibodies described herein. The expression profile and cell surface localization of 20P1F12/TMPRSS2 makes it a potential imaging reagent for metastasized disease. 20P1F12/TMPRSS2 is expressed in various prostate cancer xenograft tissues and cell lines, and is also expressed in some colon cancer cell lines. The expression status of 20P1F12/TMPRSS2 may provide information useful for localizing tumors, predicting susceptibility to advanced stage disease, and/or gauging tumor aggressiveness. 20P1F12/TMPRSS2 expression status in patient samples may be analyzed by, for example: (i) immunohistochemical analysis, (ii) in situ hybridization, (iii) RT-PCR analysis on laser capture micro-dissected samples, (iv) western blot analysis of clinical samples and cell lines, (v) tissue array analysis, (vi) in vivo imaging. Various immunological assays useful for the detection of 20P1F12/TMPRSS2 proteins are contemplated, including but not limited to various types of radioimmunoassays, enzyme-linked immunosorbent assays (ELISA), enzyme-linked immunofluorescent assays (ELIFA), immunocytochemical methods, and the like. As an example, 20P1F12/TMPRSS2 antibodies may be labeled and used as immunological imaging reagents capable of detecting prostate and colon cancer cells (e.g., in radioscintigraphic imaging methods). For radioscintigraphic in vivo imaging, radiolabeled 20P1F12/TMPRSS2 antibodies specifically reactive with extracellular epitopes of 20P1 F12/TMPRSS2 are preferred.

Assays for identifying prostate, prostate cancer or colon cancer cells comprise detecting polynucleotides corresponding to the 20P1F12/TMPRSS2 gene in a biological sample, such as serum, bone, prostate, colon and other tissues, urine, semen, cell preparations, and the like. Detectable 20P1F12/TMPRSS2 polynucleotides include, for example, a 20P1F12/TMPRSS2 gene or fragments thereof, 20P1F12/TMPRSS2 mRNA, alternative splice variant 20P1F12/TMPRSS2 mRNAs, and recombinant DNA or RNA molecules containing a 20P1F12/TMPRSS2 polynucleotide. A number of methods for amplifying and/or detecting the presence of 20P1 F12/TMPRSS2 polynucleotides are well known in the art and may be employed in the practice of this aspect of the invention.

In one embodiment, a method for detecting a 20P1F12/TMPRSS2 mRNA in a biological sample comprises producing cDNA from the sample by reverse transcription using at least one primer; amplifying the cDNA so produced using a 20P1F12/TMPRSS2 polynucleotides as sense and antisense primers to amplify 20P1 F12/TMPRSS2 cDNAs therein; and detecting the presence of the amplified 20P1F12/TMPRSS2 cDNA. In another embodiment, a method of detecting a 20P1 F12/TMPRSS2 gene in a biological sample comprises first isolating genomic DNA from the sample; amplifying the isolated genomic DNA using 20P1F12/TMPRSS2 polynucleotides as sense and antisense primers to amplify the 20P1 F12/TMPRSS2 gene therein; and detecting the presence of the amplified 20P1F12/TMPRSS2 gene. Any number of appropriate sense and antisense probe combinations may be designed from the nucleotide sequence provided for 20P1F12/TMPRSS2 (FIG. 1; SEQ ID NO. XX) and used for this purpose.

In another embodiment, a method of detecting the presence of a 20P1F12/TMPRSS2 protein in a biological sample comprises first contacting the sample with a 20P1F12/TMPRSS2 antibody, a 20P1F12/TMPRSS2-reactive fragment thereof, or a recombinant protein containing an antigen binding region of a 20P1F12/TMPRSS2 antibody; and then detecting the binding of 20P1F12/TMPRSS2 protein in the sample thereto.

Methods for identifying a cell which expresses 20P1F12/TMPRSS2 are also provided. In one embodiment, an assay for identifying a cell which expresses a 20P1F12/TMPRSS2 gene comprises detecting the presence of 20P1F12/TMPRSS2 mRNA in the cell. Methods for the detection of particular mRNAs in cells are well known and include, for example, hybridization assays using complementary DNA probes (such as in situ hybridization using labeled 20P1F12/TMPRSS2 riboprobes, Northern blot and related techniques) and various nucleic acid amplification assays (such as RT-PCR using complementary primers specific for 20P1F12/TMPRSS2, and other amplification type detection methods, such as, for example, branched DNA, SISBA, TMA and the like). Alternatively, an assay for identifying a cell which expresses a 20P1F12/TMPRSS2 gene comprises detecting the presence of 20P1F12/TMPRSS2 protein in the cell or secreted by the cell. Various methods for the detection of proteins are well known in the art and may be employed for the detection of 20P1F12/TMPRSS2 proteins and 20P1F12/TMPRSS2 expressing cells.

Determining the status of 20P1F12/TMPRSS2 expression patterns in an individual may be used to diagnose cancer and may provide prognostic information useful in defining appropriate therapeutic options. Similarly, the expression status of 20P1F12/TMPRSS2 may provide information useful for predicting susceptibility to particular disease stages, progression, and/or tumor aggressiveness. Therefore, another aspect of the invention provides methods and assays for determining 20P1F12/TMPRSS2 expression status and diagnosing cancers which express 20P1F12/TMPRSS2.

In one embodiment, an assay useful in determining the presence of cancer in an individual comprises detecting a significant increase in 20P1F12/TMPRSS2 mRNA or protein expression in a test cell or tissue sample relative to expression levels in the corresponding normal cell or tissue. The presence of 20P1F12/TMPRSS2 mRNA in a colon sample, for example, may indicate the emergence, presence and/or severity of colon cancer, since normal colon does not express 20P1F12/TMPRSS2. In a related embodiment, 20P1F12/TMPRSS2 expression status may be determined at the protein level rather than at the nucleic acid level. For example, such a method or assay would comprise determining the level of 20P1F12/TMPRSS2 protein expressed by cells in a test tissue sample and comparing the level so determined to the level of 20P1F12/TMPRSS2 expressed in a corresponding normal sample. The presence of 20P1F12/TMPRSS2 protein may be evaluated, for example, using immunohistochemical methods. 20P1F12/TMPRSS2 antibodies or binding partners capable of detecting 20P1F12/TMPRSS2 protein expression may be used in a variety of assay formats well known in the art for this purpose.

Peripheral blood may be conveniently assayed for the presence of prostate or colon cancer cells, using RT-PCR to detect 20P1F12/TMPRSS2 expression therein. The presence of RT-PCR amplifiable 20P1F12/TMPRSS2 mRNA may indicate the presence of one of these cancers. RT-PCR detection assays for tumor cells in peripheral blood are currently being evaluated for use in the diagnosis and management of a number of human solid tumors. In the prostate cancer field, these include RT-PCR assays for the detection of cells expressing PSA and PSM (Verkaik et al., 1997, Urol. Res. 25: 373-384; Ghossein et al., 1995, J. Clin. Oncol. 13: 1195-2000; Heston et al., 1995, Clin. Chem. 41: 1687-1688). RT-PCR assays are well known in the art.

In another approach, a recently described sensitive assay for detecting and characterizing carcinoma cells in blood may be used (Racila et al., 1998, Proc. Natl. Acad. Sci. USA 95: 4589-4594). This assay combines immunomagnetic enrichment with multiparameter flow cytometric and immunohistochemical analyses, and is highly sensitive for the detection of cancer cells in blood, reportedly capable of detecting one epithelial cell in 1 ml of peripheral blood.

Methods for detecting and quantifying the expression of 20P1F12/TMPRSS2 mRNA or protein are described herein and use standard nucleic acid and protein detection and quantification technologies well known in the art. Standard methods for the detection and quantification of 20P1F12/TMPRSS2 mRNA include in situ hybridization using labeled 20P1F12/TMPRSS2 riboprobes, Northern blot and related techniques using 20P1F12/TMPRSS2 polynucleotide probes, RT-PCR analysis using primers specific for 20P1F12/TMPRSS2, and other amplification type detection methods, such as, for example, branched DNA, SISBA, TMA and the like. In a specific embodiment, semiquantitative RT-PCR may be used to detect and quantify 20P1F12/TMPRSS2 mRNA expression as described in the Examples which follow. Any number of primers capable of amplifying 20P1F12/TMPRSS2 may be used for this purpose, including but not limited to the various primer sets specifically described herein. Standard methods for the detection and quantification of protein may be used for this purpose. In a specific embodiment, polyclonal or monoclonal antibodies specifically reactive with the 20P1F12/TMPRSS2 protein may be used in an immunohistochemical assay of biopsied tissue.

We also describe kits for the diagnostic and therapeutic applications described or suggested above. Such kits may comprise a carrier means being compartmentalized to receive in close confinement one or more container means such as vials, tubes, and the like, each of the container means comprising one of the separate elements to be used in the method. For example, one of the container means may comprise a probe which is or can be detectably labeled. Such probe may be an antibody or polynucleotide specific for 20P1F12/TMPRSS2 protein or gene/mRNA, respectively. Where the kit utilizes nucleic acid hybridization to detect the target nucleic acid, the kit may also have containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radionucleotide label.

### EXAMPLES

### EXAMPLE 1: ISOLATION OF cDNA CORRESPONDING TO 20P1F12/TMPRSS2 GENE BY SSH HYBRIDIZATION CLONING AND EXPRESSION ANALYSIS

### MATERIALS AND METHODS

### Cell lines and Human Tissues

All human cancer cell lines used in this study were obtained from the ATCC. All cell lines were maintained in DMEM with 10% fetal calf serum. PrEC (primary prostate epithelial cells) were obtained from Clonetics and were grown in PrEBM media supplemented with growth factors (Clonetics).

All human prostate cancer xenografts were originally provided by Charles Sawyers (UCLA) (Klein et al., 1997). LAPC-4 AD and LAPC-9 AD xenografts were routinely passaged as small tissue chunks in recipient SCID males. LAPC-4 Al and LAPC-9 Al xenografts were derived as described previously (Klein et al., 1997) and were passaged in castrated males or in female SCID mice.

Human tissues for RNA and protein analyses were obtained from the Human Tissue Resource Center (HTRC) at the UCLA (Los Angeles, CA) and from QualTek, Inc. (Santa Barbara, CA). A benign prostatic hyperplasia tissue sample was patient-derived.

### RNA Isolation:

Tumor tissue and cell lines were homogenized in Trizol reagent (Life Technologies, Gibco BRL) using 10 ml/ g tissue or 10 ml/ 10⁸ cells to isolate total RNA. Poly A RNA was purified from total RNA using Qiagen's Oligotex mRNA Mini and Midi kits. Total and mRNA were quantified by spectrophotometric analysis (O.D. 260/280 nm) and analyzed by gel electrophoresis.

### Oligonucleotides:

**The following HPLC purified oligonucleotides were used.**

### RSACDN (cDNA synthesis primer):

**5'TTTTGTACAAGCTT₃₀3'**

### Adaptor 1:

5'CTAATACGACTCACTATAGGGCTCGAGCGGCCGCCCGGGCAGGT3'
3'GGCCCGTCCA5'

### Adaptor 2:

5'GTAATACGACTCACTATAGGGCAGCGTGGTCGCGGCCGAGGT3'
3'CGGCTCCA5'

### PCR primer 1:

**5'CTAATACGACTCACTATAGGGC3'**

### Nested primer (NP)1:

**5'TCGAGCGGCCGCCCGGGCAGGT3'**

### Nested primer (NP)2:

**5'AGCGTGGTCGCGGCCGAGGT3'**

### Suppression Subtractive Hybridization:

Suppression Subtractive Hybridization (SSH) was used to identify cDNAs corresponding to genes which may be up-regulated in androgen dependent prostate cancer compared to benign prostatic hyperplasia.

Double stranded cDNAs corresponding to the LAPC-4 AD xenograft (tester) and the BPH tissue (driver) were synthesized from 2 µg of poly(A)* RNA isolated from xenograft and BPH tissue, as described above, using CLONTECH's PCR-Select cDNA Subtraction Kit and 1 ng of oligonucleotide RSACDN as primer. First- and second-strand synthesis were carried out as described in the Kit's user manual protocol (CLONTECH Protocol No. PT1117-1, Catalog No. K1804-1). The resulting cDNA was digested with Rsa I for 3 hrs. at 37°C. Digested cDNA was extracted with phenol/chloroform (1:1) and ethanol precipitated.

Driver cDNA (BPH) was generated by combining in a 4 to 1 ratio Rsa I digested BPH cDNA with digested cDNA from mouse liver, in order to ensure that murine genes were subtracted from the tester cDNA (LAPC-4 AD).

Tester cDNA (LAPC-4 AD) was generated by diluting 1 µl of Rsa I digested LAPC-4 AD cDNA (400 ng) in 5 µl of water. The diluted cDNA (2 µl, 160 ng) was then ligated to 2 µl of adaptor 1 and adaptor 2 (10 µM), in separate ligation reactions, in a total volume of 10 µl at 16°C overnight, using 400 u of T4 DNA ligase (CLONTECH). Ligation was terminated with 1 µl of 0.2 M EDTA and heating at 72°C for 5 min.

The first hybridization was performed by adding 1.5 µl (600 ng) of driver cDNA to each of two tubes containing 1.5 µl (20 ng) adaptor 1- and adaptor 2- ligated tester cDNA. In a final volume of 4 µl, the samples were overlayed with mineral oil, denatured in an MJ Research thermal cycler at 98°C for 1.5 minutes, and then were allowed to hybridize for 8 hrs at 68°C. The two hybridizations were then mixed together with an additional 1 µl of fresh denatured driver cDNA and were allowed to hybridize overnight at 68°C. The second hybridization was then diluted in 200 µl of 20 mM Hepes, pH 8.3, 50 mM NaCl, 0.2 mM EDTA, heated at 70°C for 7 min. and stored at -20°C.

### PCR Amplification, Cloning and Sequencing of Gene Fragments Generated from SSH:

To amplify gene fragments resulting from SSH reactions, two PCR amplifications were performed. In the primary PCR reaction 1 µl of the diluted final hybridization mix was added to 1 µl of PCR primer 1 (10 µM), 0.5 µl dNTP mix (10 µM), 2.5 µl 10 x reaction buffer (CLONTECH) and 0.5 µl 50 x Advantage cDNA polymerase Mix (CLONTECH) in a final volume of 25 µl. PCR 1 was conducted using the following conditions: 75°C for 5 min., 94°C for 25 sec., then 27 cycles of 94°C for 10 sec, 66°C for 30 sec, 72°C for 1.5 min. Five separate primary PCR reactions were performed for each experiment. The products were pooled and diluted 1:10 with water. For the secondary PCR reaction, 1 µl from the pooled and diluted primary PCR reaction was added to the same reaction mix as used for PCR 1, except that primers NP1 and NP2 (10 µM) were used instead of PCR primer 1. PCR 2 was performed using 10-12 cycles of 94°C for 10 sec, 68°C for 30 sec, 72°C for 1.5 minutes. The PCR products were analyzed using 2% agarose gel electrophoresis.

The PCR products were inserted into pCR2.1 using the T/A vector cloning kit (Invitrogen). Transformed E. coli were subjected to blue/white and ampicillin selection. White colonies were picked and arrayed into 96 well plates and were grown in liquid culture overnight. To identify inserts, PCR amplification was performed on 1 ml of bacterial culture using the conditions of PCR1 and NP1 and NP2 as primers. PCR products were analyzed using 2% agarose gel electrophoresis.

Bacterial clones were stored in 20% glycerol in a 96 well format. Plasmid DNA was prepared, sequenced, and subjected to nucleic acid homology searches of the GenBank, dBest, and NCI-CGAP databases.

### RT-PCR Expression Analysis:

First strand cDNAs were generated from 1 µg of mRNA with oligo (dT)12-18 priming using the Gibco-BRL Superscript Preamplification system. The manufacturers protocol was used and included an incubation for 50 min at 42°C with reverse transcriptase followed by RNAse H treatment at 37°C for 20 min. After completing the reaction, the volume was increased to 200 µl with water prior to normalization. First strand cDNAs from 16 different normal human tissues were obtained from Clontech.

Normalization of the first strand cDNAs from multiple tissues was performed by using the primers 5'atatcgccgcgctcgtcgtcgacaa3' and 5'agccacacgcagctcattgtagaagg 3' to amplify β-actin. First strand cDNA (5 µl) was amplified in a total volume of 50 µl containing 0.4 µM primers, 0.2 µM each dNTPs, 1XPCR buffer (Clontech, 10 mM Tris-HCL, 1.5 mM MgCl₂, 50 mM KCl, pH8.3) and 1X Klentaq DNA polymerase (Clontech). Five µ of the PCR reaction was removed at 18, 20, and 22 cycles and used for agarose gel electrophoresis. PCR was performed using an MJ Research thermal cycler under the following conditions: initial denaturation was at 94°C for 15 sec, followed by a 18, 20, and 22 cycles of 94°C for 15, 65°C for 2 min, 72°C for 5 sec. A final extension at 72°C was carried out for 2 min. After agarose gel electrophoresis, the band intensities of the 283 bp β-actin bands from multiple tissues were compared by visual inspection. Dilution factors for the first strand cDNAs were calculated to result in equal β-actin band intensities in all tissues after 22 cycles of PCR. Three rounds of normalization were required to achieve equal band intensities in all tissues after 22 cycles of PCR.

To determine expression levels of the 20P1F12 gene, 5 µl of normalized first strand cDNA was analyzed by PCR using 25, 30, and 35 cycles of amplification using the following primer pairs, which were designed with the assistance of (MIT; for details, see, www.genome.wi.mit.edu):

5' AGT CTT CCT GCT GAG TCC TTT CC 3'
5' CAA GGG CAC TGT CTA TAT TCT CAC C 3'

Semi quantitative expression analysis was achieved by comparing the PCR products at cycle numbers that give light band intensities.

### RESULTS:

Several SSH experiments were conduced as described in the Materials and Methods, supra, and led to the isolation of numerous candidate gene fragment clones. All candidate clones were sequenced and subjected to homology analysis against all sequences in the major public gene and EST databases in order to provide information on the identity of the corresponding gene and to help guide the decision to analyze a particular gene for differential expression.

One of the cDNA clones, designated 20P1 F12, showed identity to a recently described serine protease TMPRSS2 (Paoloni-Giacobino et al., 1997, Genomics 44: 309-320). The isolated 20P1F12 cDNA fragment is 388 bp in length and has the nucleotide sequence shown in FIG. 4. Differential expression analysis by RT-PCR showed that the 20P1F12 gene is expressed at approximately equal levels in normal prostate and the LAPC-4 and LAPC-9 xenografts (FIG. 5, panel A). Further RT-PCR expression analysis of first strand cDNAs from 16 normal tissues showed greatest levels of 20P1F12 expression in prostate. Substantially lower level expression was observed in several other normal tissues (i.e., colon, pancreas, kidney, liver and lung) (FIG. 5, panels B and C).

### EXAMPLE 2: NORTHERN BLOT ANALYSIS OF 20P1F12/TMPRSS2 GENE EXPRESSION

Northern blot analysis on a panel of 16 normal human tissues using a labeled 20P1 F12/TMPRSS2 probe (corresponding to the 20P1 F12 SSH cDNA of FIG. 4) were conducted to confirm the prostate specificity of 20P1F12/TMPRSS2 expression initially established by RT-PCR expression analysis. The results, shown in FIG. 6 (Panels A & B), confirm and extend the RT-PCR analyses and show that 20P1F12/TMPRSS2 expression is relatively prostate specific, as expression in prostate is clearly many times greater than expression in lung, kidney, pancreas or colon, where only very low level expression is detected. No detectable expression was observed in any of the other 11 normal tissues used in this panel.

In addition, 20P1F12/TMPRSS2 expression levels in the LAPC-4 and LAPC-9 xenografts were also examined by Northern blot analysis. The results, shown in FIG. 6 (Panel C), indicate similar expression levels in the xenografts and normal tissue, with lower level expression seen in the LAPC-9 xenograft only. Further Northern blot analysis of 20P1F12/TMPRSS2 expression in a large panel of cancer cells is described in Example 4, below.

### EXAMPLE 3: CLONING OF FULL LENGTH 20P1F12 cDNA

A full length cDNA encoding the 20P1F12/TMPRSS2 gene was isolated from a human prostate library and designated 20P1F12-GTC1. The nucleotide and amino acid sequences of 20P1F12-GTC1 are shown in FIG 1. Plasmid p20P1F12-GTC1 (carrying the 20P1F12-GTC1 cDNA) was deposited with the ATCC (Manassas, Virginia) on February 12, 1999 and has been accorded ATCC Designation Number 207097. The approximately 3.5 kb 20P1 F12-GTC1 cDNA encodes a protein of 492 amino acids which is almost, but not completely, identical to the sequence previously described (FIG. 2). There are several differences in the nucleotide sequence of the 20P1F12-GTC1 cDNA relative to the published TMPRSS2 sequence, five of which result in different encoded amino acids, as shown in the amino acid alignment of FIG. 3. Specifically, four of the amino acid differences are in the protease domain, three of which are non-conservative amino acid differences which could affect protease function and/or specificity. It is unclear how these amino acid sequence differences might affect biological activity. However, it is possible that 20P1F12/TMPRSS2 and TMPRSS2 are differentially expressed in view of applicants' data showing divergent mRNA expression pattern in normal human tissues.

### EXAMPLE 4: 20P1F12/TMPRSS2 EXPRESSION IN PROSTATE AND COLON CANCER

To analyze 20P1F12/TMPRSS2 expression in cancer tissues and cell lines, Northern blotting was performed on RNA derived from the LAPC xenografts and a panel of prostate and non-prostate cancer cell lines. The results show high levels of 20P1F12/TMPRSS2 expression in all the LAPC xenografts and in colon cancer cell lines (FIG. 7). Similar expression levels were detected in prostate, LAPC-4 AD, LAPC-4 Al, LAPC-9 AD and LNCaP. Lower levels of 20P1 F12/TMPRSS2 were seen in LAPC-9 Al and PC-3 cells with no expression seen in DU145. High levels of 20P1F12/TMPRSS2 expression were also detected in three out of four colon cancer cell lines, including LoVo, T84 and Colo-205.

### EXAMPLE 5: CHARACTERIZATION OF 20P1F12/TMPRSS2 PROTEIN

### Generation of 20P1F12/TMPRSS2 Monoclonal Antibodies

TMPRSS2 represents a potential therapeutic target for prostate and colon cancers. As a cell surface antigen, it may be a particularly good target for antibody therapy. To explore this possibility and to further characterize the 20P1F12/TMPRSS2 protein, monoclonal antibodies directed against a GST-20P1F12/TMPRSS2 fusion protein were generated. The immunogen comprised an approximately 8 kD region within the protease domain, specifically amino acid residues 362 through 440 (see FIG. 1). Mice were immunized with purified GST-TMPRSS2 and hybridomas were generated. Hybridoma supernatants were screened for specific antibodies by western blotting using lysates from 293T cells transfected with 20P1F12/TMPRSS2. A total of 6 hybridomas were identified that specifically recognize 20P1F12/TMPRSS2 by Western blotting (FIG. 8a).

Western blotting of LNCaP, LAPC-4 and LAPC-9 cell lysates identifies two major protein bands of approximately 70 and 32 kilodaltons (kD) (FIG. 8b). The predicted molecular weight (MW) of 20P1F12/TMPRSS2 is 54 kD, suggesting that the 70 kD isoform is modified, possibly by glycosylation. The 32 kD form may be a proteolytically cleaved fragment containing the carboxyl-terminal epitopes recognized by the antibodies.

Additional 20P1F12/TMPRSS2 mAbs may be generated by cell-based immunization using LAPC-9 cells and PC-3 cells expressing 20P1F12/TMPRSS2 as a screening agent for cell-based ELISAs. In addition, 20P1 F12/TMPRSS2 mAbs may be generated using purified 20P1F12/TMPRSS2 protein as immunogen. For example, recombinant 20P1F12/TMPRSS2 having an amino-terminal His-tag may be expressed in a baculovirus system using pBlueBac4.5 (Invitrogen). His-tagged 20P1F12/TMPRSS2 may then be purified using a Nickel column, quantified and used as immunogen. Screening of monoclonal may be performed using cell-based ELISAs with, for example, LNCaP and PC-3/TMPRSS2 cells.

### Cell Surface Localization

To study the characteristics of the 20P1 F12/TMPRSS2 protein, 20P1F12 cDNA (FIG. 1) was cloned into pcDNA 3.1 Myc-His (Invitrogen), which provides a 6-His tag at the carboxyl-terminus. The construct was transfected into 293T cells and was analyzed by cell-surface biotinylation. Biotinylated cell surface proteins were affinity purified using streptavidin-sepharose. Western blot analysis of streptavidin affinity purified proteins using an anti-His antibody demonstrated the presence of 20P1F12/TMPRSS2 protein (FIG. 9a). Therefore, as predicted from sequence analysis, 20P1F12/TMPRSS2 is expressed at the cell surface of transfected cells.

To examine cell surface expression of endogenous 20P1F12/TMPRSS2 in LNCaP and PC-3 prostate cancer cells, biotinylated cell surface proteins were affinity purified with streptavidin-sepharose and probed with anti-20P1F12/TMPRSS2 antibodies. Western blotting of streptavidin purified proteins clearly show cell surface biotinylation of endogenous 20P1F12/TMPRSS2 in both LNCaP and PC-3 cells appearing as 32 and 70 kD protein bands (FIG. 9b). In additional controls, 20P1F12/TMPRSS2 protein was not detected in streptavidin precipitates from non-biotinylated cells (Fig. 8b). This data combined with sequence analysis predict 20P1F12/TMPRSS2 to be a type II transmembrane protein.

Interestingly, 293T cells transfected with a carboxyl-terminal His-tagged 20P1F12/TMPRSS2 express primarily the 70 kD protein (FIG. 9a). Since the 20P1 F12/TMPRSS2 protease domain is located at the carboxyl-terminus, it is possible that the 32 kD fragment is a result of auto-catalytic cleavage, which is inhibited by the His tag. The related molecule, hepsin (TMPRSS1), appears to be capable of autoactivation in a concentration dependent manner (Vu et al., 1997, J. Biol. Chem. 272: 31315-31320). This auto-catalytic cleavage may be exploited to identify small molecules that inhibit 20P1F12/TMPRSS2 activity. Cells may be grown in the presence or absence of small molecule inhibitors to specifically look for inhibition of cleavage. Such small molecules may be tested as prostate cancer therapeutics.

### Glycosylation of 20P1F12/TMPRSS2

The predicted MW of 20P1 F12/TMPRSS2 is significantly smaller than the apparent MW detected by Western blotting. This suggests that 20P1F12/TMPRSS2 may be glycosylated. The GTC1 sequence indicates that there are three potential glycosylation sites with the consensus sequence of NXS/T (residues 128, 213, 249). To explore the possibility that 20P1F12/TMPRSS2 is glycosylated, His-tagged 20P1F12/TMPRSS2 was transfected into 293T cells and purified using a Nickel-agarose (Invitrogen). Affinity purified protein was eluted with 50 mM EDTA, pH 8.0, and was de-glycosylated using N-glycosidase F (Boehringer Mannheim) according to the manufacturers protocol. Untreated and de-glycosylated protein were analyzed by western blotting using anti-His antibodies. The results show a 5-8 kD MW shift of 20P1F12/TMPRSS2 with N-glycosidase F treatment (FIG 10), indicating that 20P1F12/TMPRSS2 is indeed a glycosylated protein. De-glycosylated 20P1F12/TMPRSS2 still exhibited a MW of at least 5-10 kD larger than the predicted size, indicating that either the de-glycosylation reaction was not complete (or that glycosylation is O-linked), or that 20P1F12/TMPRSS2 may exhibit additional post-translational modifications (such as phosphorylation, sulfation).

### Androgen Regulation

Northern blotting shows that expression of 20P1F12/TMPRSS2 seems to decrease in the androgen independent LAPC-9 xenograft and the androgen independent cell lines PC-3 and DU145 (FIG. 6), suggesting that 20P1F12/TMPRSS2 may be an androgen regulated gene. To explore this possibility, LNCaP cells, which are androgen dependent and express significant levels of 20P1F12/TMPRSS2, were deprived of androgen for one week by growing them in media containing 2% charcoal-stripped fetal bovine serum (FBS). The cells were then stimulated with mibolerone, a synthetic androgen analogue, at various time points. Cells were harvested for RNA and Northern blotting. As a loading control, the same blot was also probed with β-actin. The results (FIG. 11) show a clear reduction of 20P1P12/TMPRSS2 expression during androgen deprivation (FIG. 11). Addition of mibolerone increased 20P1F12/TMPRSS2 expression significantly, indicating that it is an androgen responsive gene. Expression of prostate-specific antigen (PSA) in the same samples was monitored as a positive control for androgen regulation (FIG. 11).

To determine the optimal time of 20P1F12/TMPRSS2 induction, androgen starved cells were stimulated with mibolerone for various time points. Cells were harvested for RNA and protein isolation to perform northern and western blotting respectively. The results (FIG. 12) show induction of 20P1F12/TMPRSS2 message within three hours of stimulation and increased through 24 hours after hormone addition.

To analyze the protein levels, western blotting of cell lysates using the 1 F9 mAb was performed. Additional controls for 20P1F12/TMPRSS2 expression included PC-3 cells infected with a retrovirus encoding either neo or 20P1F12/TMPRSS2. Infected PC-3 cells were selected in G418 for 2-3 weeks and harvested for western blotting. The results showed strong expression of 20P1F12/TMPRSS2 in the cells infected with a 20P1F12/TMPRSS2 virus, and no detectable 20P1F12/TMPRSS2 expression in the neo cells.

When looking at androgen deprived LNCaP cells, 20P1 F12/TMPRSS2 expression is still detectable, but visibly reduced when compared to androgen stimulated cells. However, the first time point of induced expression appears after 9 hours of stimulation, indicating that protein expression of 20P1F12/TMPRSS2 lags behind RNA induction (FIG. 12).

These results demonstrate that 20P1F12/TMPRSS2 is an androgen regulated gene, similar to other prostate specific proteases, such as PSA and hK2 (Young et al., 1995, J. Androl. 16:97).

### Effect of 20P1F12/TMPRSS2 on NIH 3T3 Morphology

20P1F12/TMPRSS2 exhibits prostate specific expression and seems to be regulated by androgen. To determine the effect of expressing 20P1P12/TMPRSS2 in a heterologous non-prostate cancer cell line, 20P1 F12/TMPRSS2 retrovirus was used to infect NIH 3T3 cells. The morphology of cells infected with 20P1F12/TMPRSS2 retrovirus was compared to the morphology of control (neo) virus infected cells. A population of infected cells exhibited a distinct vacuolar appearance compared to control cells (FIG. 13), which seem to correlate with high levels of expression. Upon passaging this infected cell population, vacuole-bearing cells gradually disappeared with apparently reduced expression of 20P1 F12/TMPRSS2.

### Evaluation of 20P1F12/MPRSS2 Function

20P1 F12/TMPRSS2 function may be being assessed in mammalian cells engineered to express 20P1F12/TMPRSS2. For this purpose, 20P1F12/TMPRSS2 is conveniently cloned into several vectors, including pcDNA 3.1 myc-His-tag (Invitrogen), the retroviral vector pSRαtkneo (Muller et al., 1991, MCB 11:1785), and pIND (Invitrogen) an ecdysone-inducible expression system. Using these expression vectors, 20P1F12/TMPRSS2 is expressed in several cell lines, including PC-3, NIH 3T3, mouse L cell fibroblasts and 293T. Expression of 20P1F12/TMPRSS2 is monitored using anti-20P1F12TMPRSS2 antibodies by Western and FACS analysis. Purified TMPRSS2 may used to identify the substrate.

Such mammalian cell lines expressing 20P1F12/TMPRSS2 are then tested in several in vitro, including cell proliferation, cell adhesion, cell invasion using a membrane invasion culture system (MICS) (Welch et al. ,Int. J. Cancer 43: 449-457) in tissue culture, and in vivo assays, including tumor formation in SCID mice. The 20P1F12/TMPRSS2 cell phenotype is compared to the phenotype of cells which do not express 20P1F12/TMPRSS2.

To assess the functional role of the different domains in 20P1F12/TMPRSS2, the following deletion mutants and point mutants are generated: (i) ΔSRCR (93 a.a. deletion); (ii) ΔLDLRA (35 a.a. deletion); and (iii) mutant of the catalytic triad: H296Q, D345N, S441A (single point mutants). 20P1F12/TMPRSS2 mutants are cloned into the retroviral vectors psRαtkneo for expression in mammalian cells. The resulting mutants are useful for elucidating the importance of the different domains and residues. In addition, these experiments are useful for determining whether such mutants function as dominant negative molecules. Dominant negative activity may be manifested in cells that express endogenous 20P1F12/TMPRSS2, such as LNCaP. Dominant negative activity may be due to interactions with substrates via protease domain, or via the protein-protein interaction domains. The mutant 20P1 F12/TMPRSS2 molecules are tested in the same in vitro and in vivo assays as wild-type 20P1F12/TMPRSS2 (see above). Such dominant negative 20P1F12/TMPRSS2 molecules may be useful therapeutically. For example, a dominant negative 20P1F12/TMPRSS2 may introduced into prostate cancer cells via gene therapy vectors capable of delivering and expressing the corresponding coding sequence into prostate tumor cells. Similarly, such methods may be useful in the treatment of colon cancer.

Determining the characteristics of 20P1F12/TMPRSS2 expression in normal mouse tissues and in transgenic mice provides further information about the function of 20P1F12/TMPRSS2. Northern blot analysis using probes designed from the 20P1F12/TMPRSS2 sequences provided herein may be used to define the expression pattern of murine 20P1F12/TMPRSS2. In addition, 20P1F12/TMPRSS2 expression during development in the mouse embryo can be analyzed. The resulting data will identify a tissue source for cloning the mouse gene and predict which tissues would be affected in a transgenic mouse knock-out study.

transgenic mouse may be generated and used to define the biological role of 20P1F12/TMPRSS2 in an in-vivo setting. In one approach, the human or mouse 20P1F12/TMPRSS2 genes are used to generate transgenic mice. Over-expression of spontaneous tumor formation in mice may be studies using transgenic mice. In another approach, 20P1F12/TMPRSS2 gene knock-outs are generated in mice. Such mice may also be crossed with other prostate cancer mouse models, such as the TRAMP model (Greenberg et al., 1995, PNAS 92:3439) to study the influence on prostate cancer aggressiveness and metastasis and to observe changes in disease progression.

Experiments testing 20P1F12/TMPRSS2 functional interaction with serine protease inhibitors will also provide information on 20P1F12/TMPRSS2 function. For this purpose, inhibition is accomplished using small molecule inhibitors or biological inhibitors.

## Claims

1. An isolated 20P1F12/TMPRSS2 protein comprising the amino acid sequence shown in Fig. 1.

2. An isolated polynucleotide selected from (a) a polynucleotide having the nucleotide sequence as shown in Fig. 1, wherein T can also be U; (b) a polynucleotide encoding a 20P1F12/TMPRSS2 polypeptide whose sequence is encoded in the cDNA contained in plasmid p20P1F12-GTC1 as deposited with American Type Culture Collection as Accession No. 207097; and (c) a polynucleotide encoding the 20P1F12/TMPRSS2 protein of Fig. 1.

3. A recombinant expression vector which contains a polynucleotide according to claim 2.

4. The expression vector of claim 3, wherein the vector is the plasmid p20P1F12-GTC1 as deposited with American Type Culture Collection as Accession No. 207097.

5. The expression vector of claim 3, wherein the vector is a viral vector.

6. The expression vector of claim 5, wherein the viral vector is selected from vaccinia, fowlpox, canarypox, adenovirus, influenza, poliovirus, adeno-associated virus, lentivirus and Sindbis virus.

7. A host cell which contains an expression vector according to any one of claims 3 to 6.

8. An assay for determining the presence of prostate or colon cancer in an individual, comprising contacting a test sample of prostate or colon tissue with an antibody that immunospecifically binds to the protein consisting of the amino acid sequence of Fig. 1, and which antibody is labeled with a detectable marker, and detecting the binding of 20P1F12/TMPRSS2 protein in the sample thereto, wherein a significant increase in 20P1F12/TMPRSS2 protein expression in the test tissue sample relative to expression levels in the corresponding normal tissue indicates the presence of prostate or colon cancer.

9. An assay for determining the presence of prostate or colon cancer in an individual, comprising:
(a) contacting a sample of prostate or colon tissue with a polynucleotide probe which specifically hybridizes to the polynucleotide of Fig. 1; and
(b) detecting the presence of a hybridization complex formed by the hybridization of the probe with 20P1F12/TMPRSS2 polynucleotide in the sample, wherein the presence of the hybridization complex indicates the presence of 20P1F12/TMPRSS2 polynucleotide within the sample, wherein a significant increase in 20P1F12/TMPRSS2 polynucleotide in the sample relative to levels in a corresponding normal tissue indicates the presence of cancer.

10. An assay for determining the presence of prostate or colon cancer in an individual, comprising:
(a) producing cDNA from a prostate or colon tissue sample by reverse transcription using at least one primer;
(b) amplifying the cDNA so produced by using sense and antisense primers to amplify 20P1F12/TMPRSS2 cDNAs therein; and
(c) detecting the presence of the amplified 20P1F12/TMPRSS2 cDNA,
wherein the sense and antisense primers are capable of amplifying the polynucleotide of Fig. 1, and wherein a significant increase in 20P1F12/TMPRSS2 mRNA or cDNA in the test tissue sample relative to levels in a corresponding normal tissue indicates the presence of prostate or colon cancer.

11. An assay according to any one of claims 8 to 10, wherein the cancer is prostate cancer.

12. An antibody or antigen binding fragment thereof that immunospecifically binds to the protein consisting of the amino acid sequence of Fig. 1, for use in a method for the treatment of prostate cancer.

13. An antibody or antigen binding fragments of claim 12 conjugated to a toxin or therapeutic agent for use in a method for the treatment of prostate cancer.

14. Use of an antibody or antigen binding fragment thereof that immunospecifically binds to the protein consisting of the amino acid sequence of Fig. 1, for the manufacture of a medicament for the treatment of prostate cancer.

15. Use according to claim 14, wherein the antibody or fragment thereof is conjugated to a toxin or therapeutic agent.

## Patentansprüche

1. Isoliertes 20PF12/TMPRSS2-Protein mit der in Fig. 1 dargestellten Aminosäuresequenz.

2. Isoliertes Polynucleotid, ausgewählt unter (a) einem Polynucleotid mit der in Fig. 1 dargestellten Nucleotidsequenz, wobei T auch U sein kann; (b) einem Polynucleotid, das für ein 20P1F12/TMPRSS2-Polypeptid codiert, dessen Sequenz in der cDNA codiert ist, die in dem bei der American Type Culture Collection unter der Zugriffsnummer 207097 hinterlegten Plasmid p20P1F12-GTC 1 enthalten ist; und (c) einem Polynucleotid, das für das 20P1F12/TMPRSS2-Protein von Fig. 1 codiert.

3. Rekombinanter Expressionsvektor, der ein Polynucleotid nach Anspruch 2 enthält.

4. Expressionsvektor nach Anspruch 3, wobei der Vektor das bei der American Type Culture Collection unter der Zugriffsnummer 207097 hinterlegte Plasmid p20P1F12-GTC1 ist.

5. Expressionsvektor nach Anspruch 3, wobei der Vektor ein viraler Vektor ist.

6. Expressionsvektor nach Anspruch 5, wobei der virale Vektor unter Kuhpocken, Geflügelpocken, Kanarienpocken, Adenovirus, Influenza, Poliovirus, einem adenoasssoziertem Virus, Lentivirus und Sindbis-Virus ausgewählt ist.

7. Wirtszelle, die einen Expressionsvektor nach einem der Ansprüche 3 bis 6 enthält.

8. Assay zur Bestimmung des Vorhandenseins von Prostata- oder Kolonkrebs in einer Person, wobei der Assay aufweist: Inkontaktbringen einer Untersuchungsprobe von Prostata- oder Kolongewebe mit einem Antikörper, der immunspezifisch an das Protein bindet, das aus der Aminosäuresequenz von Fig. 1 besteht, wobei dieser Antikörper mit einem nachweisbaren Marker markiert ist, und Nachweis, daß das Protein 20P1F12/TMPRSS2 in der Probe daran bindet, wobei eine signifikante Zunahme der Expression des Proteins 20P1F12/TMPRSS2 in der zu untersuchenden Gewebeprobe gegenüber den Expressionsgraden in entsprechendem normalem Gewebe das Vorhandensein von Prostata- oder Kolonkrebs anzeigt.

9. Assay zur Bestimmung des Vorhandenseins von Prostata- oder Kolonkrebs in einer Person, wobei der Assay aufweist:
(a) Inkontaktbringen einer Probe von Prostata- oder Kolongewebe mit einer Polynucleotid-Sonde, die spezifisch an das Polynucleotid von Fig. 1 hybridisiert; und
(b) Nachweis der Anwesenheit eines Hybridisierungskomplexes, der durch die Hybridisierung der Sonde mit dem Polynucleotid 20P1F12/TMPRSS2 in der Probe gebildet wird, wobei die Anwesenheit des Hybridisierungskomplexes die Gegenwart des Polynucleotids 20P1F12/TMPRSS2 innerhalb der Probe anzeigt, wobei eine signifikante Zunahme des Polynucleotids 20P1F12/TMPRSS2 in der Probe gegenüber Spiegeln in einem entsprechenden normalen Gewebe das Vorhandensein von Krebs anzeigt.

10. Assay zur Bestimmung des Vorhandenseins von Prostata- oder Kolonkrebs in einer Person, wobei der Assay aufweist:
(a) Erzeugen von cDNA aus einer Prostata- oder Kolongewebeprobe durch reverse Transkription unter Verwendung mindestens eines Primers;
(b) Amplifizieren der so erzeugten cDNA unter Verwendung von Sense- und Antisense-Primern, um darin 20P1F12/TMPRSS2-cDNAs zu amplifizieren; und
(c) Nachweis der Anwesenheit der amplifizierten 20P1F12/TMPRSS2-cDNA, wobei die Sense- und Antisense-Primer imstande sind, das Polynucleotid von Fig. 1 zu amplifizieren, und wobei eine signifikante Zunahme von 20P1F12/TMPRSS2-mRNA oder -cDNA in der zu untersuchenden Gewebeprobe gegenüber Speiegeln in einem entsprechenden normalen Gewebe das Vorhandensein von Prostata- oder Kolonkrebs anzeigt.

11. Assay nach einem der Ansprüche 8 bis 10, wobei der Krebs Prostatakrebs ist.

12. Antikörper oder antigenbindendes Fragment davon, der (das) immunspezifisch an das aus der Aminosäuresequenz von Fig. 1 bestehende Protein bindet, zur Verwendung bei einem Verfahren zur Behandlung von Prostatakrebs.

13. Antikörper oder antigenbindendes Fragment nach Anspruch 12, der (das) an ein Toxin oder Therapeutikum ankonjugiert ist, zur Verwendung bei einem Verfahren zur Behandlung von Prostatakrebs.

14. Verwendung eines Antikörpers oder eines antigenbindenden Fragments davon, der (das) immunspezifisch an das aus der Aminosäuresequenz von Fig. 1 bestehende Protein bindet, für die Herstellung eines Medikaments zur Behandlung von Prostatakrebs.

15. Verwendung nach Anspruch 14, wobei der Antikörper oder das Fragment davon an ein Toxin oder Therapeutikum ankonjugiert ist.

## Revendications

1. Protéine 20P1F12/TMPRSS2 isolée comprenant la séquence d'acides aminés montrée dans la Fig. 1.

2. Polynucléotide isolé choisi parmi (a) un polynucléotide possédant la séquence de nucléotides telle que montrée dans la Fig. 1, dans laquelle T peut également être U; (b) un polynucléotide codant pour un polypeptide 20P1F12/TMPRSS2 dont la séquence est codée dans l'ADNc contenu dans le plasmide p20P1F12-GTC1 tel que déposé avec la collection de cultures types américaine sous le No. d'entrée 207097; et (c) un polynucléotide codant pour la protéine 20P1F12/TMPRSS2 de la Fig. 1.

3. Vecteur d'expression recombinant qui contient un polynucléotide selon la revendication 2.

4. Vecteur d'expression selon la revendication 3, où le vecteur est le plasmide p20P1F12-GTC1 tel que déposé avec la collection de cultures types américaine sous le No. d'entrée 207097.

5. Vecteur d'expression selon la revendication 3, où le vecteur est un vecteur viral.

6. Vecteur d'expression selon la revendication 5, où le vecteur viral est choisi parmi la vaccine, la variole aviaire, la variole du canari, un adénovirus, le virus de la grippe, le virus de la poliomyélite, un virus associé aux adénovirus, un lentivirus et le virus de Sindbis.

7. Cellule hôte qui contient un vecteur d'expression selon l'une quelconque des revendications 3 à 6.

8. Dosage pour la détermination de la présence d'un cancer de la prostate ou du côlon chez un individu, comprenant la mise en contact d'un échantillon d'essai d'un tissu de prostate ou de côlon avec un anticorps qui se lie immuno-spécifiquement à la protéine constituée de la séquence d'acides aminés de la Fig. 1, et lequel anticorps est marqué avec un marqueur détectable, et la détection de la liaison de la protéine 20P1F12/TMPRSS2 dans l'échantillon à celui-ci, dans lequel une augmentation significative dans l'expression de la protéine 20P1F12/TMPRSS2 dans l'échantillon d'essai de tissu relativement aux niveaux d'expression dans le tissu normal correspondant indique la présence d'un cancer de la prostate ou du côlon.

9. Dosage pour la détermination de la présence d'un cancer de la prostate ou du côlon chez un individu, comprenant:
(a) la mise en contact d'un échantillon d'un tissu de prostate ou de côlon avec une sonde de polynucléotide qui s'hybride spécifiquement au polynucléotide de la Fig. 1; et
(b) la détection de la présence d'un complexe d'hybridation formé par l'hybridation de la sonde avec le polynucléotide 20P1F12/TMPRSS2 dans l'échantillon, dans lequel la présence du complexe d'hybridation indique la présence du polynucléotide 20P1F12/TMPRSS2 dans l'échantillon, dans lequel une augmentation significative dans le polynucléotide 20P1F12/TMPRSS2 dans l'échantillon relativement aux niveaux dans un tissu normal correspondant indique la présence d'un cancer.

10. Dosage pour la détermination de la présence d'un cancer de la prostate ou du côlon chez un individu, comprenant:
(a) la production d'un ADNc à partir d'un échantillon d'un tissu de prostate ou de côlon par une transcription inverse en utilisant au moins une amorce;
(b) l'amplification de l'ADNc ainsi produit en utilisant des amorces sens et anti-sens pour amplifier les ADNc de 20P1F12/TMPRSS2 à l'intérieur; et
(c) la détection de la présence de l'ADNc de 20P1F12/TMPRSS2 amplifié, dans lequel les amorces sens et anti-sens sont capables d'amplifier le polynucléotide de la Fig. 1 et dans lequel une augmentation significative dans un ARNm ou un ADNc de 20P1F12/TMPRSS2 dans l'échantillon d'essai de tissu relativement aux niveaux dans un tissu normal correspondant indique la présence d'un cancer de la prostate ou du côlon.

11. Dosage selon l'une quelconque des revendications 8 à 10, dans lequel le cancer est un cancer de la prostate.

12. Anticorps ou fragment se liant à un antigène de celui-ci qui se lie immuno-spécifiquement à la protéine constituée de la séquence d'acides aminés de la Fig. 1, pour une utilisation dans une méthode pour le traitement d'un cancer de la prostate.

13. Anticorps ou fragment se liant à un antigène selon la revendication 2 conjugué à une toxine ou un agent thérapeutique pour une utilisation dans une méthode pour le traitement d'un cancer de la prostate.

14. Utilisation d'un anticorps ou d'un fragment se liant à un antigène de celui-ci qui se lie immuno-spécifiquement à la protéine constituée de la séquence d'acides aminés de la Fig. 1, pour la fabrication d'un médicament pour le traitement d'un cancer de la prostate.

15. Utilisation selon la revendication 14, dans laquelle l'anticorps ou un fragment de celui-ci est conjugué à une toxine ou un agent thérapeutique.
